(19) Europäisches Patentamt
European Patent Office
Office européen des brevets

(11) **EP 1 882 446 B1**

(12) **EUROPÄISCHE PATENTSCHRIFT**

(45) Veröffentlichungstag und Bekanntmachung des Hinweises auf die Patenterteilung:
**12.11.2008 Patentblatt 2008/46**

(51) Int Cl.:
***A61B 5/00*** *(2006.01)*

(21) Anmeldenummer: 06015332.7

(22) Anmeldetag: **24.07.2006**

(54) **Vorrichtung zur Messung eines Analyten in einer Augenflüssigkeit**

Device for measuring an analyte in an ocular fluid

Dispositif pour la mesure d'un analyte dans un fluide oculaire

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**30.01.2008 Patentblatt 2008/05**

(73) Patentinhaber: EyeSense AG
**4051 Basel (CH)**

(72) Erfinder:
• **Müller, Achim**
**63762 Grossostheim (DE)**
• **Herbrechtsmeier, Peter**
**61462 Königstein (DE)**

• **Haberstroh, Klaus**
**78351 Bodman-Ludwigshafen (DE)**
• **Gruler, Roman**
**78628 Rottweil (DE)**

(74) Vertreter: **Isenbruck, Günter**
**Isenbruck, Bösl, Hörschler, Wichmann, Huhn Patentanwälte**
**Theodor-Heuss-Anlage 12**
**68165 Mannheim (DE)**

(56) Entgegenhaltungen:
**EP-A1- 0 868 881          WO-A-20/04071287**
**US-A- 6 122 042**

**Beschreibung**

Gebiet der Erfindung

Die Erfindung betrifft ein Handmessgerät zur Messung mindestens eines Analyten in einer Augenflüssigkeit sowie ein analytisches Messsystem, welches das erfindungsgemäße Handmessgerät beinhaltet. Weiterhin wird ein Verfahren offenbart zur Bestimmung einer Konzentration mindestens eines Analyten in einer Körperflüssigkeit, welches das analytische Messsystem einsetzt. Derartige Handmessgeräte, analytische Messsysteme und Verfahren werden beispielsweise zur Bestimmung von Blutglukosekonzentration eingesetzt.

Stand der Technik

Die Bestimmung der Blutglukosekonzentration sowie eine entsprechende Medikation ist für Diabetiker ein essentieller Bestandteil des Tagesablaufs. Dabei muss die Blutglukosekonzentration schnell und einfach mehrmals am Tage, typischerweise 2- bis 7-mal bestimmt werden, um gegebenenfalls entsprechende medizinische Maßnahmen ergreifen zu können. In vielen Fällen erfolgt dabei die Medikation mittels automatischer Systeme, insbesondere mit Insulinpumpen.

Herkömmliche Systeme zur Bestimmung der Blutglukosekonzentration basieren in der Regel darauf, dass der Patient oder ein Arzt, beispielsweise mittels eines geeigneten Lanzettensystems, einen Hautbereich perforiert und dadurch eine Blutprobe generiert. Diese Probe wird anschließend mittels geeigneter Messverfahren, beispielsweise optischer und/oder elektrochemischer Messverfahren, auf ihren Blutglukosegehalt analysiert.

Um die mit der häufigen Generierung von Blutproben verbundenen Unannehmlichkeiten der Patienten zu verringern, wurden verschiedene nicht-invasive oder minimal-invasive Technologien zur Messung von Blutglukosekonzentrationen entwickelt. Eine Technologie basiert auf der Messung von Glukose in Augenflüssigkeiten, wie beispielsweise Tränenflüssigkeit, Kammerwasser oder interstitieller Flüssigkeit. So ist beispielsweise in WO 01/13783 ein Okularsensor für Glukose beschrieben, welcher als Augenlinse ausgestaltet ist. Der Okularsensor umfasst einen Glukoserezeptor, welcher mit einem ersten Fluoreszenzlabel markiert ist, und einen Glukose-Konkurrenten, welcher mit einem zweiten Fluoreszenzlabel ("Donor") markiert ist. Die beiden Fluoreszenzlabel sind derart ausgewählt, dass, wenn der Konkurrent an den Rezeptor gebunden ist, die Fluoreszenz des zweiten Fluoreszenzlabels aufgrund eines resonanten Fluoreszenz-Energietransfers gelöscht wird (Quenching). Durch Überwachen der Veränderung der Fluoreszenzintensität bei einer Wellenlänge um das Fluoreszenzmaximum des quenchbaren Fluoreszenzlabels kann der Anteil des Fluoreszenz-markierten Konkurrenten gemessen werden, welcher durch die Glukose verdrängt worden ist. Auf diese Weise kann die Glukosekonzentration in der Augenflüssigkeit bestimmt werden. Diese Messung kann wiederum genutzt werden, um daraus auf die Blutglukosekonzentration zu schließen. Auch andere Arten von Nachweisen sind denkbar und dem Fachmann geläufig, beispielsweise ein Fluoreszenznachweis des ersten Fluoreszenzlabels.

Auch WO 02/087429 beschreibt ein Fluoreszenz-Photometer, mittels dessen Blutglukosekonzentrationen durch Messung der Glukosekonzentration in einer Augenflüssigkeit bestimmt werden können. Die dargestellte Vorrichtung ist in der Lage, gleichzeitig zwei Fluoreszenzintensitäten eines Donors bei zwei verschiedenen Wellenlängen zu messen.

Die Messung von Glukose oder anderen Analyten in Augenflüssigkeiten ist üblicherweise durch verschiedene Faktoren limitiert. Ein Faktor besteht beispielsweise darin, dass die Augenflüssigkeiten üblicherweise nur in geringen Mengen zur Verfügung stehen (wie beispielsweise Tränen- oder interstitielle Flüssigkeiten) oder nur schwer zugänglich sind (Glaskörper-Flüssigkeit oder Kammerwasser). Somit stellt in der Regel die Möglichkeit, diese Augenflüssigkeiten als Probe zu sammeln, eine sehr schwierige Vorgehensweise dar. Um diese Einschränkung beziehungsweise Schwierigkeit zu umgehen oder zu verringern, wurden verschiedene Möglichkeiten der in-vivo-Messung entwickelt. Die bereits genannte WO 01/13783 zeigt ein derartiges in-vivo-Messsystem.

Eine Schwierigkeit dieser in-vivo-Messsystems besteht jedoch darin, dass in vielen Fällen eine exakte Positionierung des Messgerätes relativ zum Auge beziehungsweise relativ zu der eingesetzten Augenlinse eine kritische Voraussetzung darstellt, von welcher die Genauigkeit der Messung entscheidend abhängt.

Daher zeigt WO 2004/071287 ein Fluoreszenz-Photometer, welches mittels zweier verschiedener Strahlen arbeitet und eine korrekte Positionierung des Messgerätes vor dem Auge ermöglicht. Mittels eines Pilotstrahls wird eine erste Fluoreszenz der Pupille angeregt, aus welcher ein Abstand zwischen dem Fluoreszenz-Photometer und dem Auge ermittelt wird. Bei Einstellung eines korrekten Abstandes wird automatisch ein Messstrahl gestartet, welcher im Auge eine zweite Fluoreszenz des Analytsensors anregt, aus welcher wiederum die Analytkonzentration bestimmt werden kann.

Trotz des erheblichen Messaufwandes, mit welchem das in WO 2004/071287 gezeigte System verbunden ist, hat sich herausgestellt, dass die Messung der Analytkonzentration nach wie vor hohen Schwankungen unterworfen ist. Zudem sind in vielen Fällen selbständig durch den Patienten durchgeführte Positionierungsvorgänge erforderlich, welche gerade von älteren Patienten oder Kindern nur schwer durchführbar sind.

Eine Vorrichtung gemäß dem Oberbegriff von Anspruch 1 ist aus der US 6,122,042 bekannt.

Aufgabe der Erfindung

Aufgabe der vorliegenden Erfindung ist es daher, eine Vorrichtung zur Verfügung zu stellen, mittels derer die Konzentration des Analyten in der Augenflüssigkeit zuverlässig, schnell und einfach bestimmt werden kann, wobei die beschriebenen Nachteile der aus dem Stand der Technik bekannten Vorrichtungen vermieden werden sollen.

Beschreibung der Erfindung

Diese Aufgabe wird durch die Erfindung mit den Merkmalen des unabhängigen Anspruchs gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht.

Die Erfindung beruht im Wesentlichen auf der Erkenntnis, dass die Reproduzierbarkeit der Messung nicht nur, wie beispielsweise in WO 2004/071287 beschrieben, vom Abstand eines Messgerätes zum Auge abhängt, sondern in vielen Fällen auch von einer Winkelstellung des Messgerätes relativ zur Blickrichtung und/oder von einer Winkelausrichtung des Messgerätes (Drehung, Verkippung) relativ zum Auge. Ein der Erfindung zugrundeliegender Gedanke besteht somit darin, bekannte opthalmische diagnostische Messgeräte zur Bestimmung der Analytkonzentration mit Positionserkennungs- und/oder Positionierungssystemen zu kombinieren, welche bislang lediglich aus dem Bereich der Prozessautomatisierung oder Robotik bekannt sind.

Es wird dementsprechend ein Handmessgerät zur Messung mindestens eines Analyten in einer Augenflüssigkeit eines Auges vorgeschlagen, welcher ein Messsystem und ein Positionierungssystem umfasst. Bei den Augenflüssigkeiten kann es sich beispielsweise um die eingangs beschriebenen Flüssigkeiten handeln. Bei dem mindestens einen Analyten kann es sich zum Beispiel um Glukose und/oder um ein Hormon oder auch um andere Arten von Analyten handeln.

Das Messsystem und das Positionierungssystem sind hier funktionell definiert, so dass es sich bei diesen Systemen nicht notwendigerweise um getrennte Systeme handeln muss. Einzelne Funktionen dieser Systeme können beispielsweise auch ganz oder teilweise von denselben Komponenten oder auch ganz oder teilweise von einem Computer, beispielsweise einem programmtechnisch (z. B. mittels entsprechender Softwaremodule) eingerichteten Mikrocomputer, gleichzeitig übernommen werden.

Das Messsystem ist eingerichtet, um mindestens eine Eigenschaft des Analyten selbst und/oder mindestens eine Analyt-bedingte Eigenschaftsänderung mindestens eines Okularsensors in der Augenflüssigkeit zu messen. Das Positionierungssystem ist eingerichtet, um eine räumliche Positionierung zu messen.

Diese räumliche Positionierung umfasst erfindungsgemäß nicht nur einen einfachen Abstand zwischen mindestens einem Messort des Auges und dem Handmessgerät, sondern weiterhin mindestens eine weitere Positionierungskoordinate. Diese mindestens eine weitere Positionierungskoordinate umfasst vorzugsweise mindestens eine der folgenden Größen: einen Winkel einer virtuellen Verbindungslinie zwischen dem Handmessgerät und dem mindestens einen Messort in einem vorgegebenen Winkelsystem (beispielsweise relativ zu einer Blickrichtung des Auges), eine (z. B. kartesische) Koordinate des Handmessgeräts, eine (z. B. kartesische) Koordinate des mindestens einen Messorts und/oder einen Ausrichtungswinkel (Drehung, Verkippung) des Handmessgeräts in einem vorgegebenen Koordinatensystem.

Für die Funktionsweise des Messsystems kann beispielsweise auf die eingangs genannten Druckschriften verwiesen werden. Das Messsystem ist eingerichtet, um auf den Analyt selbst zu reagieren (beispielsweise eine Fluoreszenz von Glukose) oder, alternativ oder zusätzlich, mittelbar auf die Anwesenheit des Analyten mittels eines Okularsensors in der Augenflüssigkeit. Neben den aus dem Stand der Technik bekannten Systemen werden jedoch auch weitere Messsysteme vorgeschlagen. So umfasst das Messsystem vorzugsweise mindestens eines der folgenden Systeme: ein Infrarot (IR)-spektroskopisches Messsystem, ein Nah-Infrarot (NIR)-spektroskopisches Messsystem, ein RAMAN-spektroskopisches Messsystem, ein UV/sichtbar (UV/VIS)-spektroskopisches Messsystem, ein Fluoreszenzmesssystem, ein Impedanz-Messsystem, ein photoakustisches Messsystem, ein zirkular-dichroitisches Messsystem, ein refraktometrisches Messsystem, ein interferometrisches Messsystem. Derartige Messsysteme und deren Aufbau sind dem Fachmann aus anderen Bereichen der Technik bekannt.

Erfindungsgemäß umfasst das Positionierungssystem mindestens eines der im kennzeichnenden Teil von Anspruch 1 genannten Systeme.

Beispielsweise kann das Handmessgerät so eingerichtet sein, dass das Positionierungssystem bei Erreichen mindestens einer vorgegebenen Soll-Positionierung oder eines vorgegebenen Soll-Positionierungsbereichs (zum Beispiel eines Toleranzbereichs) eine Messung des Messsystems automatisch auslöst. Alternativ oder zusätzlich kann das Handmessgerät auch so eingerichtet sein, dass, gesteuert durch das Positionierungssystem, eine räumliche Position und/oder räumliche Ausrichtung des Messsystems aktiv eingestellt wird. Beispielsweise kann dies derart erfolgen, dass, sobald das Handmessgerät gemäß den von dem Positionierungssystem gelieferten Informationen innerhalb eines vorgegebenen Toleranzbereichs positioniert ist, die weitere Einstellung der räumlichen Position und/oder räumlichen Ausrichtung des Messsystems innerhalb des Handmessgeräts durch steuerbare Stellelemente, beispielsweise elektromechanische Stellelemente, insbesondere eine Piezosteuerung, erfolgt. Als weitere, alternativ oder zusätzlich einsetzbare Möglichkeit ist vorzugsweise das Handmessgerät und insbesondere das Positionierungssystem derart eingerichtet, dass ein Feedback-Signal für einen Benutzer erzeugt werden kann, welches Informationen über die räumliche Positionierung liefert. Beispielsweise kann dies mittels eines Displays oder anderer optischer Signale oder auch mittels akustischer Signale erfolgen. Optische Signale können beispielsweise Hinweise für den Benutzer enthalten, in welche Richtung das Handmessgerät für eine optimale Positionierung zu bewegen und/oder zu drehen/verkippen ist. Diese Hinweise können beispielsweise mittels entsprechender Pfeile oder schriftlich gegeben werden.

Das Handmessgerät ist, wie eingangs beschrieben, vorzugsweise nicht nur eingerichtet, um die Konzentration des mindestens einen Analyten in der Augenflüssigkeit zu bestimmen, sondern vorzugsweise auch für eine Bestimmung der Analytkonzentration in einer anderen Körperflüssigkeit, insbesondere in Blut oder Gewebeflüssigkeit. Diese Konzentrationsbestimmung kann beispielsweise unter Berücksichtigung der von dem Positionierungssystem gelieferten Informationen erfolgen. So kann beispielsweise aus einer bekannten Winkel-, Abstands- und/oder Positionsabhängigkeit des vom Messsystem aufgenommenen Signals (beispielsweise eines Fluoreszenzsignals) unter Verwendung der bekannten Positionierung eine Korrektur der ermittelten Analytkonzentration durchgeführt werden.

Zur weiteren Steigerung der Genauigkeit der Messung kann zusätzlich ein Kalibriersystem vorgesehen sein, welches eingerichtet ist, um eine Vergleichsmessung mindestens einer Analytkonzentration in einer Körperflüssigkeit durchzuführen und/oder Messdaten einer mittels eines separaten Messgerätes durchgeführten entsprechenden Vergleichsmessung zu übernehmen. Die Ergebnisse dieser Vergleichsmessung beziehungsweise die Vergleichsmessungsdaten werden dann bei der Bestimmung der Konzentration des mindestens einen Analyten mit dem Handmessgerät berücksichtigt. Beispielsweise kann das Kalibriersystem ein handelsübliches System umfassen, welches beispielsweise eine Blutglukosekonzentration mittels eines üblichen elektrochemischen (zum Beispiel amperometrisch mit Glukoseoxidase oder Glukosedehydrogenase oder Hexokinase) oder optisch (zum Beispiel mittels photometrischer Messstreifen) bestimmt. Dieses Kalibriersystem kann in das Handmessgerät integriert werden, und das Messergebnis direkt in den Algorithmus der Glukosekonzentrationsbestimmung übernommen werden.

Die Idee des integrierten Kalibriersystems kann auch unabhängig von dem vorgeschlagenen Handmessgerät eingesetzt werden. So kann beispielsweise ein Messsystem, welches eingerichtet ist, um mindestens eine Eigenschaft des mindestens einen Analyten und/oder mindestens eine Analyt-bedingte Eigenschaftsänderung mindestens eines Okularsensors in der Augenflüssigkeit zu messen, kombiniert werden mit einem Kalibriersystem in einer der oben beschriebenen Ausgestaltungen. Das Messsystem kann dabei einer der in dieser Beschreibung dargestellten Ausgestaltungen entsprechen. Diese Integration kann unabhängig davon erfolgen, ob weiterhin ein Positionierungssystem vorgesehen ist oder nicht. Auf diese Weise kann bequem eine "indirekte" Messung des mindestens einen Analyten in der Augenflüssigkeit, bzw. nach entsprechender Umrechnung auch in einer anderen Körperflüssigkeit, mit einer "konventionellen" Messung (beispielsweise einer elektrochemischen Blutglucosemessung etc.) verglichen und kalibriert werden. Kalibriersystem und Messsystem können bequem in einem einzelnen Gerät (z.B. mit einem gemeinsamen Gehäuse) integriert werden. Ein fehlerträchtiger und aufwendiger Datenaustausch zwischen zwei Geräten (einem separaten Kalibriergerät und dem Messsystem), welcher beispielsweise manuell oder durch Austausch von Signalen erfolgen müsste, ist somit nicht erforderlich. Es können vorteilhafterweise gemeinsame Bedien- und Anzeigenelemente genutzt werden und gemeinsame Computer-Ressourcen und Speicherelemente. Auch kann beispielsweise das integrierte Gerät den Patienten (beispielsweise in regelmäßigen Abständen, nach Auftreten einer Störung oder nach einer Systemänderung) zu einer Kalibrationsmessung auffordern und die so gewonnen Kalibrationsdaten beispielsweise automatisch einsetzen, um die mit dem Messsystem gewonnenen Messdaten umzurechnen, beispielsweise direkt in eine Konzentration des mindestens einen Analyten in einer anderen Körperflüssigkeit (z.B. Glucosekonzentration in Blut). Diese Integration gestaltet sich insgesamt daher äußerst benutzerfreundlich und störungsunempfindlich.

Neben dem Handmessgerät wird weiterhin ein analytisches Messsystem zur Messung des mindestens einen Analyten in der Augenflüssigkeit vorgeschlagen. Das analytische Messsystem umfasst ein Handmessgerät gemäß einer der oben beschriebenen Ausführungen sowie weiterhin mindestens einen Okularsensor. Der mindestens eine Okularsensor ist geeignet, in Kontakt mit der Augenflüssigkeit gebracht zu werden. Dementsprechend kann der mindestens eine Okularsensor beispielsweise eine Augenlinse, insbesondere eine Kontaktlinse und/oder ein Augenimplantat aufweisen. Der mindestens eine Okularsensor ist ausgestaltet, um bei Kontakt mit dem mindestens einen Analyten mindestens eine Eigenschaft zu ändern, wobei die mindestens eine Eigenschaftsänderung mit dem Messsystem des Handmessgeräts messbar ist. Insbesondere kann der mindestens eine Okularsensor, analog zum eingangs beschriebenen Stand der Technik, mindestens einen Analyt-Rezeptor mit mindestens einem ersten Fluoreszenzlabel und mindestens einen Analyt-Konkurrenten mit mindestens einem zweiten Fluoreszenzlabel (im Folgenden auch als "Donor" bezeichnet) aufweisen. Dabei sollen der mindestens eine Analyt-Rezeptor und der mindestens eine Analyt-Konkurrent derart ausgestaltet sein, dass bei Bindung des mindestens einen Analyt-Konkurrenten an den mindestens einen Analyt-Rezeptor mindestens eine Eigenschaft des Okularsensors, insbesondere mindestens eine Fluoreszenzeigenschaft, sich ändert. Weiterhin kann vorteilhafterweise der mindestens eine Okularsensor auch noch einen oder mehrere Referenzfluorophore und/oder Referenzfarbstoffe (im Folgenden auch einfach als "Referenz" bezeichnet) aufweisen, welche ihre Eigenschaften bei Anwesenheit des mindestens einen Analyten nicht ändern. Dieser mindestens eine Referenzfluorophor und/oder Referenzfarbstoff kann beispielsweise von dem Positionierungssystem zur Analyt-unabhängigen Messung der räumlichen Positionierung eingesetzt werden (siehe auch die unten gezeigten Ausführungsbeispiele).

Alternativ oder zusätzlich kann der mindestens eine Okularsensor auch mindestens ein Gitter und/oder mindestens ein Hologramm aufweisen, welche jeweils ausgestaltet sind, um bei Kontakt mit dem mindestens einen Analyten mindestens eine Reflexionseigenschaft zu ändern. Beispielsweise kann ein Bragg-Gitter eingesetzt werden. Aus der Änderung der mindestens einen Reflexionseigenschaft, beispielsweise der Änderung des Bragg-Winkels, kann das Messsystem auf die Anwesenheit und/oder die Konzentration des mindestens einen Analyten schließen.

Um die Messung der räumlichen Positionierung durch das Positionierungssystem weiter zu verbessern und die Genauigkeit des analytischen Messsystems somit weiter zu steigern, kann das analytische Messsystem weiterhin zusätzlich mindestens einen Positionierungssensor umfassen. Dieser mindestens eine Positionierungssensor kann getrennt von dem mindestens einen Okularsensor sein (beispielsweise ebenfalls im Auge aufgenommen sein) oder Bestandteil des mindestens einen Okularsensors sein. Beispielsweise kann der mindestens eine Positionierungssensor eine Augenlinse, insbesondere eine Kontaktlinse und/oder ein Augenimplantat, aufweisen oder Bestandteil eines dieser Elemente sein.

Der mindestens eine Positionierungssensor soll ausgestaltet sein, um mindestens ein von dem Positionierungssystem wahrnehmbares Signal zu erzeugen. Bei diesem mindestens einen wahrnehmbaren Signal kann es sich beispielsweise um eine von dem Positionierungssystem wahrnehmbare Gestalt (zum Beispiel einen Umriss) oder eine Markierung handeln. Alternativ oder zusätzlich kann dieses Signal auch ein Fluoreszenzsignal, eine Magnetisierung, ein Referenzfluoreszenzsignal und/oder ein Referenzfarbsignal sein. Auch Änderungen dieser Signale können vom Positionierungssystem vorteilhafterweise erfasst werden. Auch Kombinationen der genannten Signalarten sind denkbar, beispielsweise eine Kombination aus einem Umriss (z. B. eine von einem Bilderkennungssystem erkennbare Form) und ein Farbsignal.

Das beschriebene Handmessgerät und das beschriebene analytische Messsystem können auf verschiedene Weisen eingesetzt werden. Ein bevorzugtes Verfahren zur Bestimmung einer Konzentration mindestens eines Analyten (zum Beispiel Glukose) in einer Körperflüssigkeit (zum Beispiel Blut) unter Verwendung des beschriebenen analytischen Messsystems in einer der dargestellten Ausgestaltungen weist die im Folgenden aufgeführten Verfahrensschritte auf. Dabei müssen die dargestellten Verfahrensschritte nicht notwendigerweise exakt in der dargestellten Reihenfolge durchgeführt werden, und es können auch zusätzliche, nicht aufgeführte Verfahrensschritte durchgeführt werden. Auch ist es möglich, einzelne oder mehrere Verfahrensschritte ganz oder teilweise parallel zueinander oder wiederholt durchzuführen.

Zunächst wird das Handmessgerät vor dem Auge, an welchem gemessen werden soll, grob positioniert. Anschließend wird die räumliche Positionierung bestimmt, und es wird eine Messung des Messsystems ausgelöst. Diese Verfahrensschritte können, wie oben beschrieben, unterschiedlich ausgestaltet sein, beispielsweise indem die gemessene räumliche Positionierung (wobei es sich auch um eine wiederholte Messung handeln kann) in die Auswertung der Messung des Messsystems einfließt, eine Positionierung des Handmessgeräts beeinflusst und/oder das Messsystem automatisch feinpositioniert wird.

Anschließend wird aus der mindestens einen gemessenen Eigenschaft des mindestens einen Analyten und/oder der mindestens einen gemessenen Eigenschaftsänderung des mindestens einen Okularsensors die Konzentration des mindestens einen Analyten in der Augenflüssigkeit bestimmt. Aus dieser Analytkonzentration in der Augenflüssigkeit wird dann mittels einer bekannten, beispielsweise in einem Datenspeicher abgelegten Relation, auf die Konzentration des mindestens einen Analyten in der Körperflüssigkeit geschlossen.

Weitere Verfahrensschritte können beispielsweise eine Speicherung der Daten, eine Darstellung der Messwerte auf einem Anzeigenelement (zum Beispiel einem Display), Warnfunktionen (zum Beispiel bei Überschreiten vorgegebener Grenzwerte), eine Steuerung von Medikationsgeräten (zum Beispiel einer Insulinpumpe), eine graphische Datenaufbereitung, Datenbankfunktionen und/oder einen Datenaustausch mit anderen Geräten (beispielsweise einer Insulinpumpe und/oder einem separaten Computer) umfassen. Weitere Funktionen sind möglich.

Weiterhin umfasst, wie oben beschrieben, das analytische Messsystem und insbesondere das Handmessgerät vorzugsweise verschiedene Computerfunktionen, welche beispielsweise durch einen Mikrocomputer mit den entsprechenden Ein- und Ausgabegeräten, Speichermedien und ähnlichem realisiert sein können. Dementsprechend wird weiterhin ein Computerprogramm mit Programmcode vorgeschlagen, welcher insbesondere auf einem maschinenlesbaren Träger gespeichert sein kann, wobei das Computerprogramm geeignet ist, bei Ausführung auf einem Computer oder Computer-Netzwerk die beschriebenen Verfahrensschritte des erfindungsgemäßen Verfahrens ganz oder teilweise zu unterstützen. Insbesondere können dabei die Verfahrensschritte der räumlichen Positionierungsbestimmung, der Auslösung der Messung, der Bestimmung der Konzentration des mindestens einen Analyten in der Augenflüssigkeit und/oder der Bestimmung der Konzentration des mindestens einen Analyten in der Körperflüssigkeit ganz oder teilweise mittels eines entsprechenden Computerprogramms realisiert werden. Diese Aspekte sind nicht Teil der Erfindung.

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt.

Die Ausführungsbeispiele sind in den Figuren schematisch dargestellt. Gleiche Bezugsziffern in den einzelnen Figuren bezeichnen dabei gleiche oder funktionsgleiche oder hinsichtlich ihrer Funktionen einander entsprechende Elemente.

Im Einzelnen zeigt:

Figur 1        ein analytisches Messsystem mit einem Handmessgerät zur Messung mindestens eines Analyten;

Figur 2A       ein Ausführungsbeispiel eines implantierten Okularsensors zur Verwendung in einem analytischen Messsystem;

Figur 2B       eine Detaildarstellung des Ausschnitts A der Darstellung gemäß Figur 2A;

Figur 3        eine Platzierung eines Okularsensors in einem menschlichen Auge;

Figur 4A       eine schematische Darstellung einer Positionierungsmessung eines dreidimensionalen Körpers mittels einer Kamera;

Figur 4B       ein einfaches Beispiel einer Abstandsmessung mittels geometrischer Größen;

Figur 4C       ein einfaches Ausführungsbeispiel eines stereoskopischen Systems;

Figur 5A       eine Prinzipdarstellung einer Laufzeit-basierten Positionierungsmessung;

Figur 5B       ein Beispiel einer Laufzeitmessung mittels Photomischdetektoren (PMDs);

Figur 6        ein Beispiel eines implantierten Okularsensors und eines zusätzlichen implantierten Positionierungssensors;

Figur 7        ein Beispiel eines Positionierungssensors mit Magnetisierung zur Messung einer räumlichen Positionierung; und

Figuren 8 bis 16    Ausführungsbeispiele von analytischen Messsystemen mit unterschiedlich ausgestalteten Handmessgeräten.

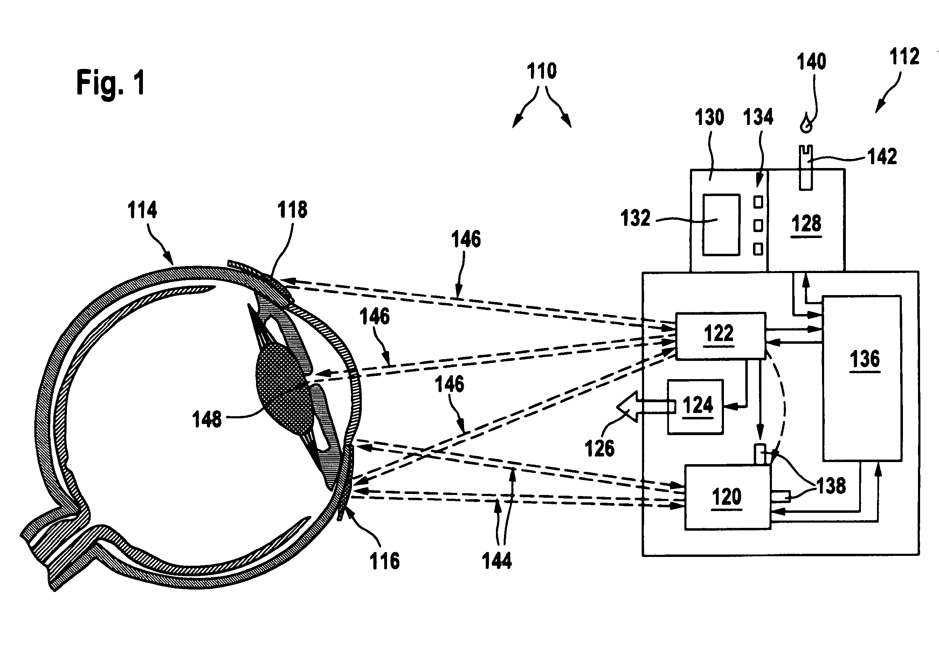

In Figur 1 ist eine schematische Prinzipdarstellung eines erfindungsgemäßen analytischen Messsystems 110 dargestellt, anhand derer die einzelnen Komponenten und deren Funktion im Folgenden erläutert werden sollen. Das analytische Messsystem 110 umfasst ein Handmessgerät 112 sowie einen in oder auf einem Auge 114 aufgenommenen Okularsensor 116 und einen Positionierungssensor 118. Wie oben erläutert, sind Okularsensor 116 und Positionierungssensor 118 optional, da das Handmessgerät 112 eine Messung beispielsweise auch anhand einer Eigenfluoreszenz des nachzuweisenden Analyten durchführen kann, und da die Positionierungsmessung auch grundsätzlich ohne Positionierungssensor 118 erfolgen kann. Weiterhin könnten auch die Funktionen von Okularsensor 116 und Positionierungssensor 118 durch ein gemeinsames Bauteil übernommen werden.

Das Handmessgerät 112 umfasst als wesentliche Komponenten ein Messsystem 120 und ein Positionierungssystem 122. Weiterhin sind in diesem schematischen Ausführungsbeispiel eine Feedback-Einheit 124 zur Erzeugung eines Feedback-Signals 126, ein Kalibriersystem 128, eine Bedieneinheit 130 mit optischen Anzeigenelementen 132 (zum Beispiel einem oder mehreren Displays) und Bedienelementen 134 (zum Beispiel Tasten, Schaltern etc.) sowie eine zentrale Recheneinheit 136 (zum Beispiel ein Mikrocomputer mit einem oder mehreren flüchtigen und/oder nichtflüchtigen Datenspeichern) vorgesehen. Die einzelnen genannten Komponenten stehen vorzugsweise untereinander in Verbindung (in Figur 1 symbolisch und ohne Anspruch auf Vollständigkeit durch Pfeile dargestellt), so dass beispielsweise eine Steuerung der Feedback-Einheit 124 und/oder des Messsystems 120 durch das Positionierungssystem 122 (direkt oder indirekt, beispielsweise über die zentrale Recheneinheit 136) vorgesehen sein kann.

Weiterhin ist in dem Ausführungsbeispiel des Handmessgeräts 112 eine Piezosteuerung 138 zur Einstellung der räumlichen Position und/oder der räumlichen Ausrichtung des Messsystems 120 vorgesehen. Das Positionierungssystem 122 nimmt (angedeutet durch den gestrichelten Pfeil) direkt oder indirekt über die zentrale Recheneinheit 136 Einfluss auf diese Piezosteuerung 138.

In dem Beispiel in Figur 1 sei angenommen, dass es sich bei dem nachzuweisenden mindestens einen Analyten um Glukose handelt. Dementsprechend ist das Kalibriersystem 128 ausgestattet, um eine "konventionelle" Vergleichsmessung einer Glukosekonzentration vorzunehmen. Dies ist in Figur 1 symbolisch durch einen Blutstropfen 140 und einen (beispielsweise elektrochemischen oder optischen) Teststreifen 142 dargestellt, wobei der Teststreifen 142 von dem Kalibriersystem 128 ausgelesen werden kann. Dem Fachmann sind auch andere mögliche Ausgestaltungen des Kalibriersystems 128 bekannt.

Weiterhin ist in Figur 1 symbolisch die Messweise des Messsystems 120 sowie des Positionierungssystems 122 dargestellt, jeweils durch gerade, gestrichelte Pfeilbündel vom Handmessgerät 112 zum Auge 114. Es sei jedoch darauf hingewiesen, dass diese Strahlenbündel keinen Anspruch auf Vollständigkeit erheben, da, wie oben dargestellt, sowohl für das Positionierungssystem 122 als auch für das Messsystem 120 verschiedene Prinzipien eingesetzt werden können. Auch muss nicht notwendigerweise, wie in Figur 1 symbolisch durch Doppelpfeile dargestellt, eine Bidirektionalität der Messungen gegeben sein, sondern es können beispielsweise auch lediglich Signale vom Auge 114 zum Handmessgerät 112 übermittelt werden, ohne dass eine Anregung durch das Handmessgerät 112 erfolgt.

Wie oben dargestellt, beruht die durch das Messsystem 120 durchgeführte Messung der Glukosekonzentration in der Augenflüssigkeit des Auges 114 entweder auf einer unmittelbaren Messung einer Eigenschaft der Glukose (beispielsweise eine Fluoreszenzeigenschaft) oder, alternativ oder zusätzlich, auf mindestens einer Analytbedingten Eigenschaftsänderung des Okularsensors 116, beispielsweise einer Änderung einer Fluoreszenzeigenschaft des Okularsensors 116 entsprechend der Glukosekonzentration. Dementsprechend verlaufen in Figur 1 symbolisch die "Messsignale" 144 zwischen dem Messsystem 120 und dem Okularsensor 116 und/oder anderen Bereichen des Auges 114. Entsprechend der verwendeten Messmethode können die Messsignale 144 beispielsweise einen oder mehrere (d.h. zum Beispiel mehrere Wellenlängen) vom Messsystem 120 zum Auge 114 entsandte Anregungsstrahlen sowie in entgegengesetzter Richtung verlaufende Antwortstrahlen (zum Beispiel Fluoreszenzlicht) umfassen. Wie oben beschrieben, sind jedoch auch andere Arten von Messsignalen 144 möglich, aus welchen sich auf die Glukosekonzentration in einer Augenflüssigkeit des Auges 114 schließen lässt.

Entsprechend sind in Figur 1 symbolisch die "Positionierungssignale" 146 als gerade, gestrichelte Doppelpfeile zwischen Positionierungssystem 122 und Auge 114 dargestellt. Das für die Messsignale 144 Gesagte gilt hier entsprechend. Als Beispiele für Positionierungssignale sind Strahlenverläufe zwischen Positionierungssystem 122 und der Pupille 148 des Auges 114, dem Positionierungssensor 118 und dem Okularsensor 116 dargestellt. Auch andere Bereiche des Auges 114 können erfasst werden sowie beispielsweise auch bekannte Formen des Auges. Wiederum ist die Ausgestaltung der Positionierungssignale 146 als Doppelpfeile lediglich symbolisch zu verstehen, da beispielsweise auch eine unidirektionale Signalübertragung (zum Beispiel ein rein visuelles Signal) zwischen Auge 114 und Positionierungssystem 122 möglich ist. Die verschiedenen Messprinzipien, welche in dem Positionierungssystem 122 eingesetzt werden können, um eine räumliche Positionierung des Handmessgeräts 112 relativ zum Auge 114 zu bestimmen, wurden oben bereits aufgelistet und sollen hier daher nicht wiederholt werden. Einige Beispiele werden im Folgenden dargestellt.

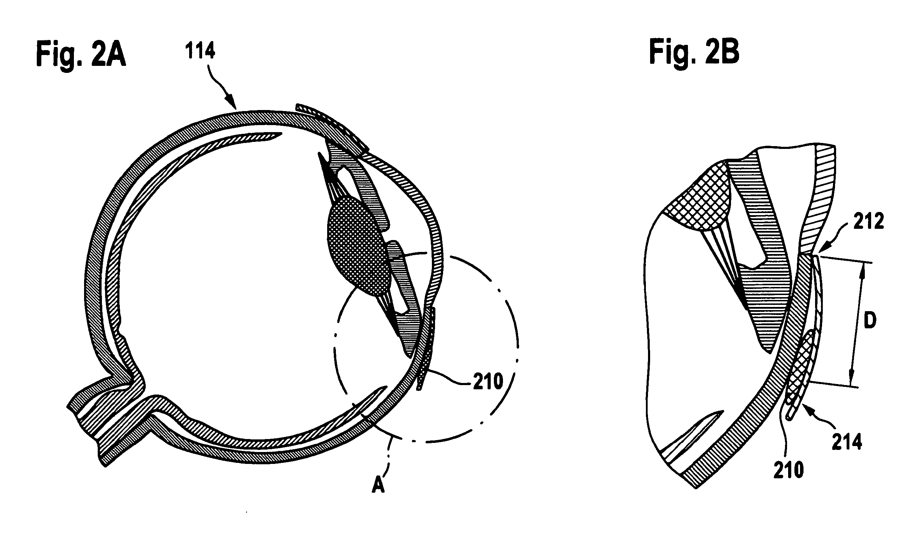

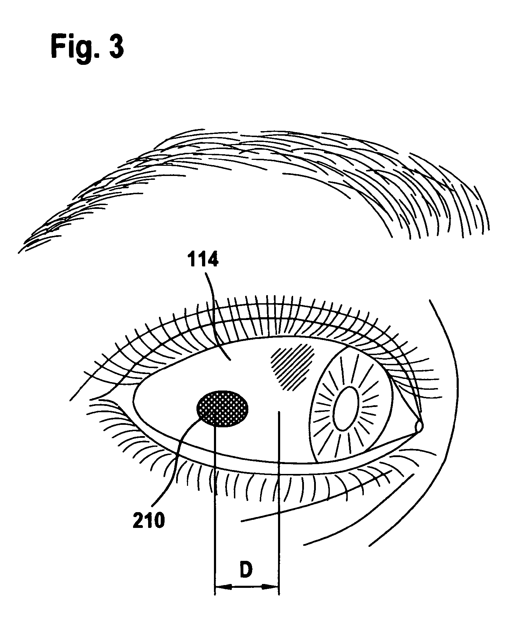

Wie oben angesprochen, basiert das analytische Messsystem 110 gemäß dem in Figur 1 dargestellten Beispiel auf einem Okularsensor 116 sowie auf einem Positionierungssensor 118. In den Figuren 2A, 2B und 3 sind Beispiele derartiger Sensoren 116, 118 dargestellt, welche als implantierte Augenlinsen ausgestaltet sind. Zu diesem Zweck sind die Augenlinsen von ihren Abmessungen und Ihrer Zusammensetzung her derart ausgebildet, dass diese implantiert werden können. Entsprechende Materialien und Dimensionen sind aus dem Stand der Technik bekannt.

Figur 2B stellt eine Detailansicht der Darstellung gemäß Figur 2A dar. Die Augenlinse 210 wird in einem Abstand D, vorzugsweise 6 mm, vom Limbus 212 unter die Conjunktiva 214 eingesetzt. Diese einfache Operation bietet beispielsweise gegenüber der Verwendung von Kontaktlinsen, welche alternativ oder zusätzlich möglich ist, den Vorteil, dass die Augenlinse 210 (Okularsensor 116 und/oder Positionierungssensor 118) ortsfest im Auge 114 untergebracht ist. Dementsprechend genauer werden die Positionierung und/oder die Messung des mindestens einen Analyten, da beide Messungen typischerweise stark von der Position der Augenlinse 210 abhängen. In der Darstellung in Figur 3 ist nochmals gezeigt, dass sich in diesem Beispiel die Augenlinse 210 in einer horizontalen Ebene mit der Pupille 148 befindet (auch andere Positionierungen sind möglich), so dass der Patient für eine Messung der Augenlinse 210 in Richtung der Nase blicken muss. Dementsprechend handelt es sich beiden Darstellungen in den Figuren 2A und 2B um Schnittdarstellungen in dieser horizontalen Ebene durch Pupille 148 und Augenlinse 210.

Als Ausführungsbeispiele des Aufbaus der Augenlinse 210 sei beispielsweise auf den in WO 01/13783 A1 beschriebenen Okularsensor verwiesen. Auch andere Ausgestaltungen sind selbstverständlich möglich. Vorteilhafterweise wird der Augenlinse 210 zusätzlich ein Referenzfarbstoff homogen eingemischt, welcher seine Fluoreszenzeigenschaften nicht mit der zu messenden Glukosekonzentration ändert. Vorteilhafterweise lässt sich dieser Referenzfarbstoff mit derselben Wellenlänge anregen, wie der auf die Glukosekonzentration empfindliche "Donor"-Farbstoff in der Augenlinse 210. Auch eine Anregung mit verschiedenen Wellenlängen ist möglich. Beispielsweise lässt sich der Farbstoff Tetramethylrhodamin (TMR) einsetzen, welcher in die Augenlinse 210 eingebracht wird. Bei der Messung durch das Messsystem 120, welches sowohl ein Signal des Donors als auch ein Referenzsignal empfangen kann, lässt sich durch eine Quotientenbildung zwischen diesen Signalen die Abhängigkeit der Messung der Analytkonzentration von Abstand zwischen Handmessgerät 112 und Auge 114 und/oder dem Winkel und/oder dem angeregten Volumen innerhalb des Auges 114 stark verringern. Dennoch besteht in der Praxis eine hohe Abhängigkeit der Messsignale und somit der Messgenauigkeit der Konzentrationsbestimmung zwischen diesem Abstand, den verschiedenen Winkeln und/oder dem Anregungsvolumen.

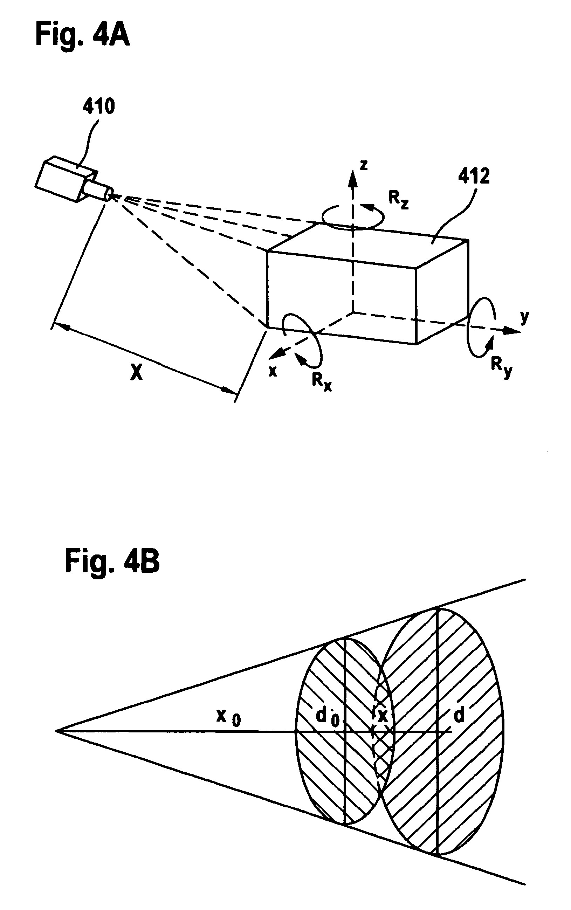

Um diese zusätzliche Abhängigkeit zu verringern, wird das in Figur 1 symbolisch dargestellte Positionierungssystem 122 eingesetzt. In den Figuren 4A bis 4C, 5A bis 5B, 6 und 7 sind verschiedene Prinzipien dargestellt, welche für eine Bestimmung der räumlichen Positionierung durch das Positionierungssystem 122 eingesetzt werden können.

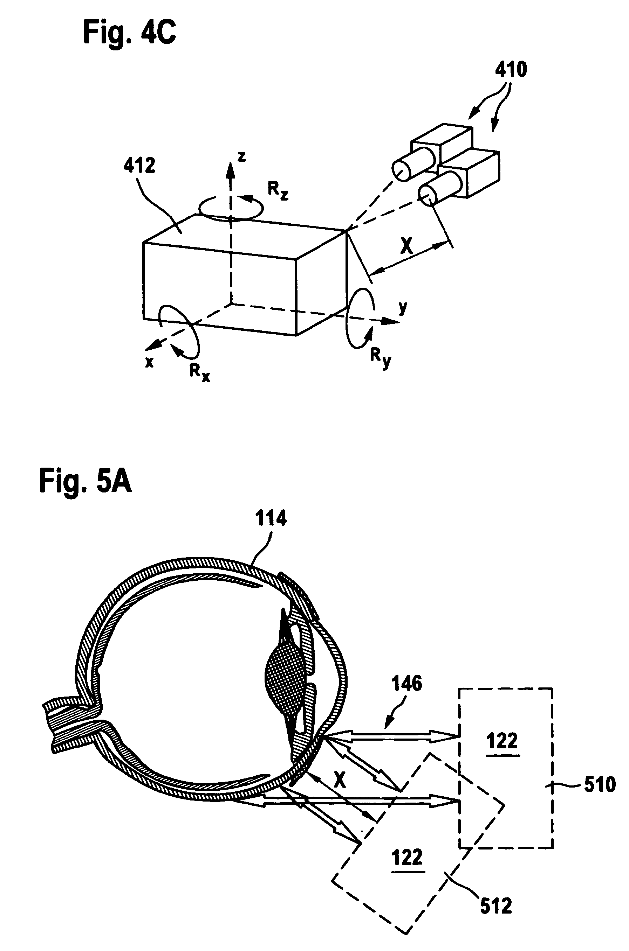

In den Figuren 4A bis 4C sind zu diesem Zweck verschiedene Kamerasysteme dargestellt. Diese Kamerasysteme umfassen mindestens eine Kamera 410, wobei das Positionierungssystem 122 in der Darstellung gemäß Figur 4A eine monokulare Kamera 410 und in der Darstellung gemäß Figur 4C eine binokulare Kamera 410 umfasst. Das binokulare Kamerasystem kann durch zwei oder mehr verschiedene Kameras 410 oder durch entsprechende Spiegelsysteme in Verbindung mit einer einzelnen Kamera realisiert werden. Derartige Systeme sind dem Fachmann bekannt.

Die in den Figuren 4A und 4B dargestellten Systeme können genutzt werden, um einen Abstand X zwischen den Kameras 410 und einem zu betrachtenden Körper 412 sowie zusätzlich weitere Positionierungskoordinaten zu erfassen. Diese Positionierungskoordinaten können, wie in den Figuren 4A und 4C symbolisch dargestellt, beispielsweise verschiedene Rotationswinkel $R_X$, $R_Y$, $R_Z$ umfassen. Dabei ist die Wahl des Koordinatensystems willkürlich und der Zweckmäßigkeit überlassen. Statt einer Rotation des Körpers 412 kann entsprechend auch eine Rotation der Kameras 410 erfasst werden, so dass beispielsweise auch der Ursprung des Koordinatensystems in die (oder eine der) Kameras 410 gelegt werden kann. Auch der Messort am Körper 412 ist vergleichsweise willkürlich und kann entsprechend festgelegt werden.

Die Kameras 410 sind vorzugsweise mit einem (in den Figuren 4A bis 4C nicht dargestellten) Bilderfassungssystem ausgestattet. Dieses Bilderfassungssystem kann beispielsweise entsprechende Kanten des Körpers 412 erkennen und dementsprechend aus diesen Kanten auf die Positionierung des Körpers 412 schließen. Zu diesem Zweck wird dem Positionierungssystem 122 beispielsweise die bekannte Gestalt des Körpers 412 vorgegeben.

In Figur 4B ist symbolisch dargestellt, wie beispielsweise mittels eines monokularen Systems gemäß der Darstellung in Figur 4A bei bekannter Größe des Körpers 412 mittels eines einfachen Strahlensatzes auf den Abstand X geschlossen werden kann. Betrachtet man zum Beispiel ein kreisrundes Sensorimplantat in Form einer Augenlinse 210, so ist das Verhältnis von Ist-Durchmesser d zu Soll-Durchmesser $d_0$ bei der Abbildung auf einem Kamera-Chip eine Funktion des aktuellen Abstandes X. Im einfachsten Fall gilt:

$$X = d \bullet X_0 / d_0.$$

Ist der Kamera-Chip beziehungsweise die Kamera 410 gegenüber der vereinfacht als Scheibe angenommenen Augenlinse 210 verkippt, so wird aus der Kreisscheibe eine Ellipse in der Abbildung. Aus dem Verhältnis von Nebenachse d' zu Hauptachse d kann beispielsweise der Kippwinkel $\alpha$ bestimmt werden zu:

$$\alpha = \frac{d'}{d}$$

Bei dem binokularen System mit den zwei Kameras 410 gemäß Figur 4C wird der Messort mit den beiden Kameras 410 aus verschiedenen Blickwinkeln betrachtet. Aus der Verschiebung der erzeugten Bilder gegeneinander kann mittels eines entsprechenden Triangulations-Algorithmus die Position und/oder Verkippung des Körpers 412 bestimmt werden. Derartige Triangulationssysteme haben gegenüber den monokularen Systemen gemäß Figur 4A den Vorteil, dass die Form oder das Abbild des Zielorts nicht notwendigerweise im Bilderfassungssystem hinterlegt sein muss.

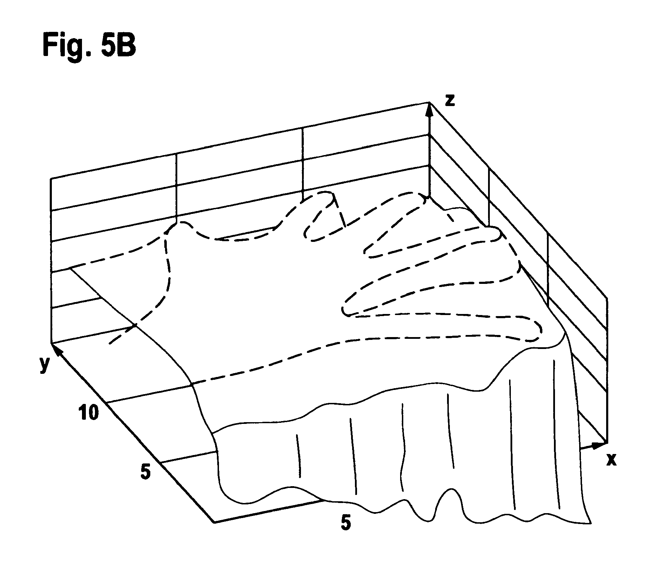

In den Figuren 5A und 5B ist ein weiteres Messprinzip dargestellt, welches vom Positionierungssystem 122 genutzt werden kann. In diesem Fall handelt es sich um ein Laufzeit-basiertes System, bei welchem von dem Positionierungssystem 122 aus Positionierungssignale 146 (zum Beispiel Laser-Pulse, akustische Signale, Infrarot-Signale oder Radar-Signale) zum Zielort am Auge 114 gesandt werden. Die Laufzeit der jeweils vom Auge 114 reflektierten Signale wird dabei gemessen. Beispielsweise kann die Messung von zwei, drei oder mehr Positionen aus erfolgen. Dementsprechend ist aus der in Figur 5A mit 510 bezeichneten ersten Position ein Unterschied in der Laufzeit zwischen den hier emittierten und reflektierten Positionierungssignalen 146 festzustellen, wohingegen in der symbolisch mit 512 bezeichneten zweiten Position die beiden (in der Praxis sind es typischerweise mehr als zwei) Positionierungssignale 146 sich in ihrer Laufzeit nicht unterscheiden. Aus der gemessenen Laufzeit sowie den Laufzeit-Unterschieden kann nicht nur der Abstand X zwischen Positionierungssystem 122 und Auge 114 bestimmt werden, sondern es kann beispielsweise auch die räumliche Ausrichtung des Positionierungssystems 122 relativ zum Auge 114 ermittelt werden.

Auch eine Messung mittels eines einzigen Positionierungssignals 146 beziehungsweise eines einzelnen Positionierungsstrahls ist möglich sowie eine Messung aus weniger als drei verschiedenen Positionen. In diesem Fall kann zur Gewinnung weiterer Informationen der Messort am Auge 114 mittels des Positionierungssignals 146 beispielsweise abgefahren werden (Scannen). Auf diese Weise lassen sich wiederum schließlich mehr als drei Positionen erzielen. Dieses Messprinzip wird kommerziell von sogenannten Photomischdetektoren (PMDs) eingesetzt. Bei PMDs wird in einem Pixel-Array, ähnlich wie bei einer CCD- oder CMOS-Kamera die Signallaufzeit (anstelle des Grauwertes bei einer Kamera) pro Pixel bestimmt und somit ein dreidimensionales Profil erstellt. Ein Beispiel einer derartigen Messung ist in Figur 5B dargestellt. Während X- und Y-Achse eine willkürliche räumliche Position darstellen, ist auf der Z-Achse die Laufzeit angegeben, welche mittels eines PMDs gemessen wurde. Auf ähnliche Weise lassen sich beispielsweise auch Konturen eines Auges erfassen, so dass nicht nur der Abstand zwischen dem Positionierungssystem 122, sondern auch zusätzlich beispielsweise Winkelstellungen des Auges 114 und/oder des Positionierungssystems 122 relativ zueinander ermitteln lassen.

Ein weiteres Messprinzip, welches vom Positionierungssystem 122 ausgenutzt werden kann, ist die Messung von Signalintensitäten. Strahlt man auf ein Objekt einen divergenten, elektromagnetischen (oder alternativ oder zusätzlich auch beispielsweise einen akustischen) Strahl, so wird ein Teil dieses Strahl divergent reflektiert. Die Intensität des divergenten reflektierten Strahls ist eine Funktion des Abstands und kann somit zur Entfernungsmessung herangezogen werden. Auch beispielsweise eine Fluoreszenzlichtquelle am Bestrahlungsort erzeugt in der Regel divergentes Fluoreszenzlicht, dessen Intensität vom Positionierungssystem 122 gemessen und zur Abstandsbestimmung herangezogen werden kann. Dieses Messprinzip zur Abstandsbestimmung kann beispielsweise genutzt werden, um die Intensität des Fluoreszenzlichts eines Referenzfluorosphors in der Augenlinse 210 zu erfassen und daraus den Abstand zwischen Positionierungssystem 122 und dem Ort der Augenlinse 210 zu bestimmen.

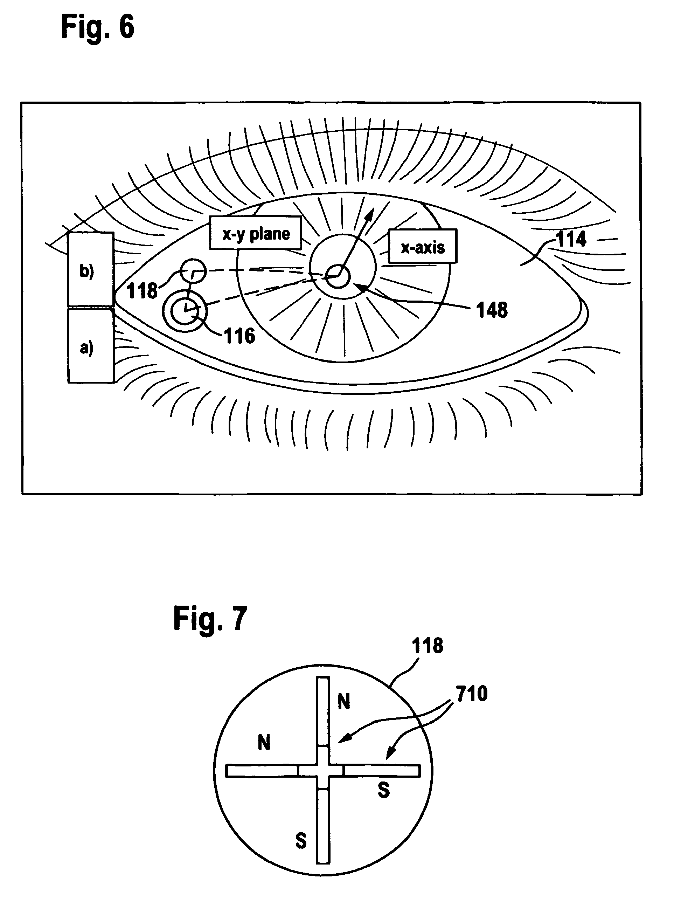

Misst man die Intensität des reflektierten Strahls und/oder der Fluoreszenz an mehr als einem Ort, so lassen sich - was durch die Erfindung genutzt wird - zusätzliche Positionierungsinformationen gewinnen. Ein Beispiel einer derartigen Messung ist in Figur 6 dargestellt. Hier wird zum einen die Fluoreszenz des Okularsensors 116 und weiterhin die Fluoreszenz des Positionierungssensors 118 sowie die Eigenfluoreszenz der Hornhaut im Bereich der Pupille 148 erfasst. Auf diese Weise lassen sich, indem die Fluoreszenzintensitäten dieser drei Orte erfasst werden, ähnlich wie bei der oben beschriebenen Laufzeitmessung, dreidimensionale Abstands- und/oder andere Positionierungsinformationen (zum Beispiel Winkel) gewinnen. Auch andere Ausgestaltungen von Intensitätsmessungen als die in Figur 6 dargestellte Ausgestaltung lassen sich einsetzen. Die Fluoreszenzintensität kann beispielsweise auch mit einer CCD-Kamera aufgenommen werden, um gleichzeitig Informationen über die Intensitäten an verschiedenen Orten zu gewinnen. Alternativ oder zusätzlich ist auch die Aufnahme mittels verschiedener Sensoren, beispielsweise unterschiedlich farbempfindlicher Sensoren möglich.

In Figur 7 ist ein weiteres Beispiel eines Messprinzips dargestellt, welches für das Positionierungssystem 122 eingesetzt werden kann. Dabei werden Magnetfeldmessungen verwendet, welche beispielsweise auf magneto-resistiven Sensoren (zum Beispiel GMR-Sensoren) beruhen. Mittels derartiger Sensoren kann die relative Position eines im Positionierungssystem 122 aufgenommenen Sensors zu einem Magnetfeld bestimmt werden. Zur Erzeugung eines derartigen Magnetfeldes dient beispielsweise der in Figur 7 dargestellte Positionierungssensor 118, welcher als separater Positionierungssensor 118 in Form einer Augenlinse 210 ins Auge 114 eingebracht werden kann, oder welcher - alternativ oder zusätzlich - auch Bestandteil des Okularsensors 116 sein kann. In dem in Figur 7 dargestellten Beispiel sind ferromagnetische oder magnetisierbare Mikro- oder Nanopartikel in einem Druckverfahren (zum Beispiel mittels Tampondruck oder ähnlichem) auf den Positionierungssensor 118 aufgedruckt. Durch gezielte Nord-/Süd-Magnetisierung 710 wird eine Messreferenz für zwei gekreuzt angeordnete magnetoresistive Sensoren im Positionierungssystem 122 erhalten, anhand derer die Winkellage zwischen den magneto-resistiven Sensoren und dem Positionierungssensor 118 bestimmt werden kann. Aus der beispielsweise insgesamt gemittelten Signalstärke ist zusätzlich auch auf den Abstand zwischen dem Positionierungssensor 118 und den magneto-resistiven Sensoren im Positionierungssystem 122 zu schließen. Auf diese Weise lassen sich Informationen über die räumliche Positionierung gewinnen.

Die oben beschriebenen Ausführungsbeispiele von Messprinzipien, auf welchen das Positionierungssystem 122 beruhen kann, sind lediglich einige Möglichkeiten, welche eingesetzt werden können. Die beschriebenen Methoden sowie weitere Methoden lassen sich in vielen Fällen auch miteinander kombinieren. Insbesondere die Kombination von Intensitätsmessungen und Kamerasystemen sowie Laufzeitmessungen und Kamerasystemen, zum Beispiel in Form von PMDs, sind geeignet zur Positionsbestimmung.

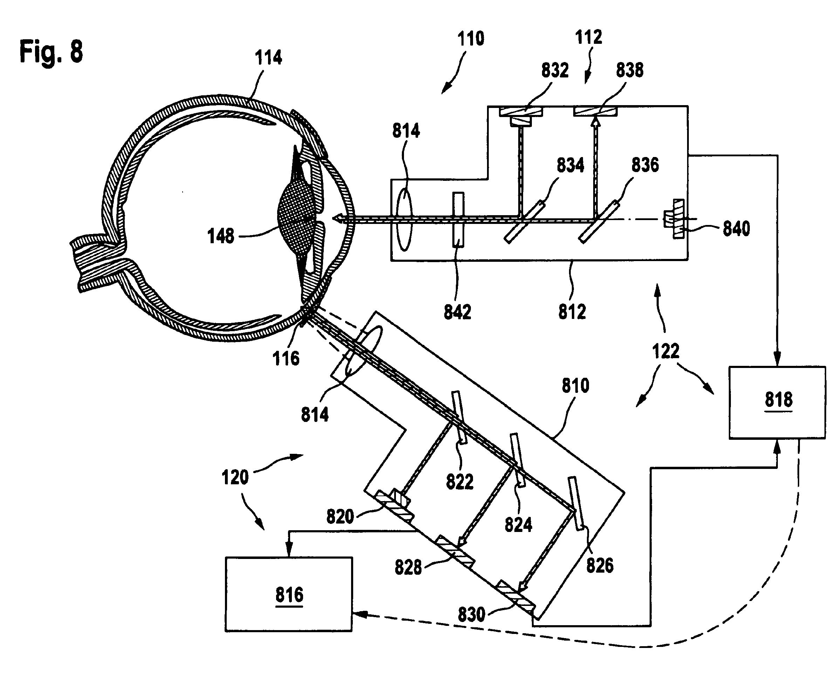

In den Figuren 8 bis 16 sind verschiedene Ausführungsbeispiele des analytischen Messsystems 110 mit einem Handmessgerät 112 dargestellt.

Bei dem in Figur 8 dargestellten Ausführungsbeispiel werden zwei getrennte optische Einheiten eingesetzt: eine Messeinheit 810 und eine Piloteinheit 812. Beide Einheiten 810, 812 sind mit einer Optik, insbesondere einer konfokalen Optik, ausgestattet, welche hier lediglich symbolisch durch die Linsen 814 angedeutet ist. Auch eine andere Ausgestaltung der Optik ist denkbar. Während die Messeinheit 810 auf den implantierten Okularsensor 116 ausgerichtet ist, ist die Piloteinheit 812 auf die Pupille 148 ausgerichtet. Die von der Messeinheit 810 generierten Informationen werden von einer Messauswertung 816 verarbeitet, wohingegen eine Positionierungsauswertung sowohl Informationen der Messeinheit 810 als auch der Piloteinheit 812 verarbeitet. Die Messauswertung 816 sowie die entsprechenden optischen Komponenten der Messeinheit 810 sind somit Bestandteile des Messsystems 120, wohingegen das Positionierungssystem 122 die Piloteinheit 812, die Positionierungsauswertung 818 sowie Teile der Messeinheit 810 umfasst.

Die Messeinheit 810 im Ausführungsbeispiel gemäß Figur 8 ist ausgestaltet, um mittels konfokaler Fluoreszenzanregung mit einer einzigen Wellenlänge für einen im Okularsensor 116 enthaltenen Donor sowie eine Referenz eine Messung der Analytkonzentration (zum Beispiel Glukose) durchzuführen. Zu diesem Zweck umfasst die Messeinheit 810 eine Anregungs-Leuchtdiode 820, welche Licht einer Wellenlänge erzeugt, mittels derer sich sowohl Donor als auch Referenzfarbstoff in dem Okularsensor 116 anregen lassen. Dieses Anregungslicht der Anregungs-Leuchtdiode 820 wird von einem auf die Anregungswellenlänge der Anregungs-Leuchtdiode 820 abgestimmten dichroitischen Spiegel 822 umgelenkt und mittels der Linse 814 (beziehungsweise einem entsprechenden optischen System) derart auf den Okularsensor 116 fokussiert, dass die Fokussierung einen kleineren Durchmesser aufweist als der Okularsensor 116 selbst. Das auf diese Weise generierte Fluoreszenzlicht des Referenzfarbstoffs beziehungsweise des auf den nachzuweisenden Analyten empfindlichen Donors wird dementsprechend vom Okularsensor 116 emittiert, von der Linse 814 gebündelt und tritt wiederum in die Messeinheit 810 ein. Der dichroitische Spiegel 822 ist derart eingerichtet, dass dieses Fluoreszenzlicht, welches üblicherweise längere Wellenlängen aufweist als das Anregungslicht der Anregungs-Leuchtdiode 820, durch diesen dichroitischen Spiegel 822 hindurchtritt, ohne reflektiert zu werden. Dementsprechend sind in der Messeinheit 810 zwei weitere dichroitische Spiegel 824 und 826 vorgesehen, welche in ihren Reflexionseigenschaften derart eingestellt sind, dass diese das Fluoreszenzlicht des Donors von denjenigen der Referenz trennen. Dementsprechend wird das Donor-Fluoreszenzlicht auf eine Donor-Photodiode 828 reflektiert, wohingegen das Referenz-Fluoreszenzlicht von dem dichroitischen Spiegel 826 auf die Referenz-Photodiode 830 reflektiert wird.

Mittels eines Vergleichs der Signale der Referenz-Photodiode und der Donor-Photodiode, gegebenenfalls in Abhängigkeit von der Anregungsintensität der Anregungs-Leuchtdiode 820 kann dann mittels der Messauswertung 816 auf die Analytkonzentration in der Augenflüssigkeit im Bereich des Okularsensors 116 geschlossen werden. Für Details dieser Messung kann beispielsweise auf WO 01/13783 A1 verwiesen werden.

Wie oben beschrieben, ist in der Regel das Ergebnis dieser Konzentrationsmessung jedoch abhängig vom Abstand des Handmessgeräts 112 vom Okularsensor 116 beziehungsweise von einer Ausrichtung des Handmessgeräts 112 relativ zum Auge 114, insbesondere einer Ausrichtung der Messeinheit 810. Zum Ausgleich dieser Ungenauigkeiten beziehungsweise zum Messen der räumlichen Positionierung dient das Positionierungssystem 122. Dabei wird in dem Aufbau gemäß Figur 8 ein Messverfahren zur Bestimmung der Positionierung verwendet, welches auf der Messung zweier Fluoreszenz-Intensitäten basiert. Als erstes, positionierungsabhängiges Intensitätssignal wird das Signal der Referenz-Photodiode 830 verwendet, welches unabhängig von der Analytkonzentration ist und lediglich abhängt vom Abstand beziehungsweise der Ausrichtung zwischen Messeinheit 810 und Auge 114. Zur Generierung eines zweiten positionierungsabhängigen Signals dient die Piloteinheit 812. Diese Piloteinheit 812 weist eine Pilot-Leuchtdiode 832 auf, welche Anregungslicht einer Wellenlänge erzeugt, die die Pupille 148 des Auges 114 zur Fluoreszenz anregt. Somit wird die Pupille 148 als zweiter Messpunkt neben dem Okularsensor 116 zur Positionierungsmessung eingesetzt.

Das Licht der Pilot-Leuchtdiode 832 wird von einem auf die Wellenlänge der Pilot-Leuchtdiode 832 abgestimmten dichroitischen Spiegel 834 umgelenkt und durch die Linse 814 in Richtung der Pupille 148 gebündelt. Von der Pupille 148 emittiertes Fluoreszenzlicht wird, da dieses üblicherweise eine längere Wellenlänge aufweist, von dem entsprechend eingestellten dichroitischen Spiegel 834 hindurchgelassen und trifft auf den weiteren, auf die Wellenlänge des Fluoreszenzlichts der Pupille abgestimmten dichroitischen Spiegel 836. Von dort wird dieses Fluoreszenzlicht auf die Pilot-Photodiode 838 gelenkt, wo dieses in ein elektrisches Signal umgewandelt wird.

Da das Signal der Pilot-Photodiode 838 von dem Abstand beziehungsweise der Ausrichtung der Piloteinheit 812 relativ zur Pupille 148 abhängt, liefert dieses Signal neben dem Signal der Referenz-Photodiode 830 eine weitere wichtige Positionierungsinformation. Aus dem Vergleich dieser beiden Intensitätssignale der Referenz-Photodiode 830 und der Pilot-Photodiode 838 kann in der Positionierungsauswertung 818 eine Positionierungsinformation gewonnen werden. Da jedes der beiden Signale der Photodioden 830 und 838 für sich genommen in eine Abstands- und/oder Winkelinformation umgerechnet werden kann, liefert die Information beider Photodioden 830, 838 zusammen mindestens eine zusätzliche Positionierungskoordinate, beispielsweise eine Winkelausrichtung.

Es sei jedoch darauf hingewiesen, dass in der Praxis eine exakte Umrechnung in Positionierungskoordinaten nicht unbedingt erforderlich ist. Vielmehr kann das Handmessgerät 112 auch in der Weise arbeiten, dass eine Positionierung, bei welcher beide Signale, also das Signal der Referenz-Photodiode 830 und das Signal der Pilot-Photodiode 838 bei vorgegebener Anregungsintensität der Dioden 820 beziehungsweise 832 eine vorgegebene Schwelle überschreiten, die Positionierung als korrekt erkannt wird. Auch "Zielkorridore" für die gemessenen Intensitäten können vorgegeben werden. Entsprechend kann beispielsweise die Positionierungsauswertung 818 direkt oder indirekt eine Messung durch die Messeinheit 810 und die Messauswertung 816 triggern.

Die Messauswertung 816 und die Positionierungsauswertung 818 können getrennte elektronische Einheiten darstellen. Sie können jedoch auch Bestandteile der in Figur 1 dargestellten zentralen Recheneinheit 136 sein und dementsprechend beispielsweise ganz oder teilweise als Software-Komponenten (zum Beispiel Programm-Module) ausgestaltet sein.

Weiterhin ist in der Piloteinheit 812 gemäß dem Ausführungsbeispiel in Figur 8 noch ein flächiges Beleuchtungselement (Backlight) 840 sowie ein transparentes Anzeigenelement 842 vorgesehen. Je nach Ausgestaltung der Piloteinheit 812 können diese unterschiedlichen Zwecken dienen und unterschiedlich ausgestaltet sein. Im einfachsten Fall kann beispielsweise das transparente Anzeigenelement 842 ein Retikel beinhalten, beispielsweise ein einfaches Fadenkreuz im Strahlengang. Mittels dieses Fadenkreuzes kann der Patient, welcher in die Piloteinheit 812 blickt, eine Grobpositionierung vornehmen. Dabei sieht der Patient, z.B. aufgrund von reflektierenden Eigenschaften des transparenten Anzeigeelements 842, der Pilot-Photodiode 838 und/oder des Backlights 840, ein Bild, in welches das Fadenkreuz eingeblendet ist. Beispielsweise kann der Patient Lichtflecke der Pilot-Leuchtdiode 832 und des Backlights 840 gleichzeitig betrachten, überlagert von dem Fadenkreuz. Dementsprechend kann er manuell eine Grobpositionierung des Handmessgeräts 112 vornehmen, derart, dass beispielsweise die Lichtflecke konzentrisch ausgerichtet sind und dass das Fadenkreuz in dieser konzentrischen Anordnung zentrisch ausgerichtet ist.

Eine andere Möglichkeit besteht darin, dass das transparente Anzeigenelement 142 zum Einblenden zusätzlicher Informationen genutzt wird. Zu diesem Zweck kann dieses beispielsweise ein Flüssigkristall-Anzeigenelement und/oder andere Anzeigenelemente beinhalten, über welche beispielsweise eine Positionierungsinformation an den Patienten übermittelt werden kann. Beispielsweise kann es sich hierbei um Pfeile handeln, welche eingeblendet werden, um zu signalisieren, dass das Handmessgerät 112 in eine entsprechende Richtung bewegt und/oder gekippt werden muss, um eine korrekte Positionierung zu erreichen. Die wiedergegebenen Informationen können beispielsweise durch das Positionierungssystem 122 generiert werden.

Bei den beschriebenen Ausgestaltungen bilden das transparente Anzeigenelement 842 sowie das Backlight 840 somit Bestandteile der Feedback-Einheit 124 (siehe Figur 1). Weitere Elemente des Handmessgeräts 112, wie beispielsweise die Bedieneinheit 130 und/oder das Kalibriersystem 128 sind in Figur 8 nicht dargestellt. Für die Funktionsweise dieser möglichen weiteren Elemente sei auf die Beschreibung zu Figur 1 verwiesen.

Als Alternative zu dem in Figur 8 dargestellten Messprinzip der Messeinheit 810 sei noch darauf hingewiesen, dass die Anregung von Donor und Referenzfluorophor im Okularsensor 116 nicht notwendigerweise mit derselben Anregungs-Leuchtdiode 820 erfolgen muss. So kann anstelle der in Figur 8 dargestellten Anordnung der Messeinheit 810 auch ein System vorgesehen sein, bei welchem neben der Anregungs-Leuchtdiode 820 für den Donor noch eine zusätzliche Anregungs-Leuchtdiode vorgesehen ist (in Figur 8 nicht dargestellt), welche speziell auf die Anregungswellenlänge des Referenzfluorophors im Okularsensor 116 abgestimmt ist. Dementsprechend könnte beispielsweise ein weiterer dichroitischer Spiegel vorgesehen sein, welcher auf die Reflexion des von dieser Referenz-Leuchtdiode emittierten Anregungslichts abgestimmt ist. Der Aufbau entspricht dem Aufbau in Figur 8, wobei lediglich ein weiterer "Arm" des in Figur 8 als 3-armiger Fächer ausgeführten Aufbaus der Messeinheit 810 hinzugefügt würde, so dass ein "4-armiger" Aufbau entstünde. Auf eine zeichnerische Darstellung sei hier verzichtet.

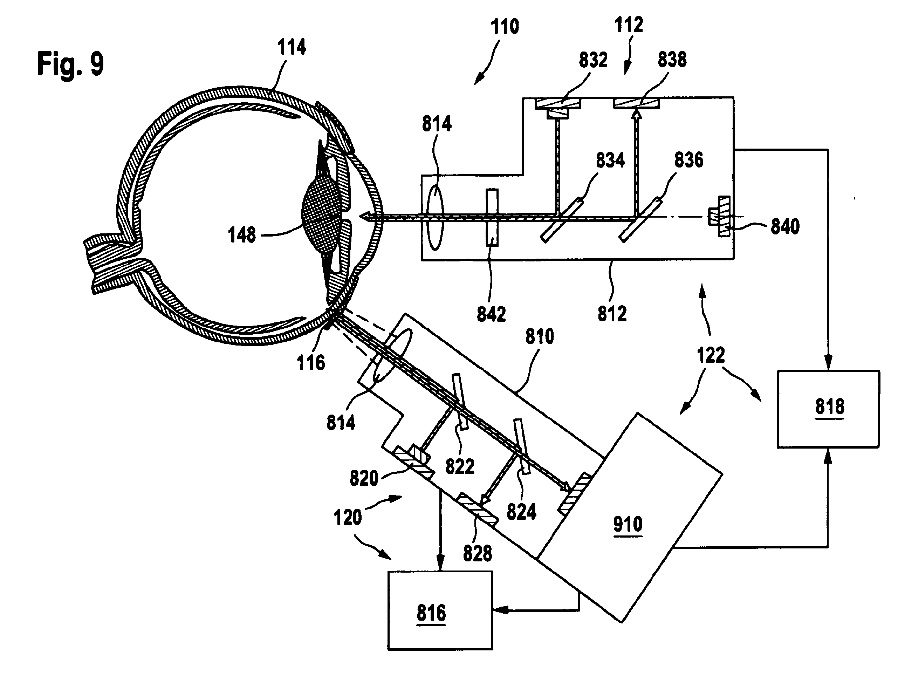

In Figur 9 ist ein zweites Ausführungsbeispiel eines analytischen Messsystems 110 mit einem Handmessgerät 112 und einem Okularsensor 116 dargestellt. Der Aufbau entspricht im Wesentlichen dem Aufbau gemäß Figur 8, so dass wiederum eine Messeinheit 810 und eine Piloteinheit 812 vorgesehen sind. Ein Unterschied zur Messeinheit 810 besteht jedoch im Ausführungsbeispiel gemäß Figur 9 darin, dass hier die Referenz-Photodiode 830 durch eine als CMOS-Kamera ausgestaltete Kamera 910 ersetzt ist. Dementsprechend kann beispielsweise auch der dichroitische Spiegel 826 entfallen, so dass Fluoreszenzlicht des Referenzfluorophors aus dem Okularsensor 116 unmittelbar in den Bildbereich der Kamera 910 projiziert wird. Alternativ kann jedoch auch ein entsprechender dichroitischer Spiegel für dieses Referenzfluoreszenzlicht vorgesehen sein.

Im Gegensatz zur Aufnahme des Referenzfluoreszenzlichts mit einer Referenz-Photodiode 830 gemäß dem Aufbau in Figur 8 wird nunmehr mittels der Kamera 910 nicht nur eine Intensitätsinformation aufgenommen, sondern ein zweidimensionales Muster (Array) von Intensitätsinformationen auf dem CMOS-Chip der Kamera 910. Auf diese Weise lässt sich, beispielsweise mittels eines (zum Beispiel in der Positionierungsauswertung 818 enthaltenen) Bildauswertungssystems noch zusätzliche räumliche Informationen gewinnen, beispielsweise entsprechend der obigen Beschreibung zu den Figuren 4A bis 4C. Alternativ oder zusätzlich lässt sich auch die Intensität über ganze Bildbereiche Mitteln.

Die Informationen dieser Kamera 910 werden der Positionierungsauswertung 818 zur Verfügung gestellt, so dass Kamera 910, Positionierungsauswertung 818 und die Piloteinheit 812 (welche analog aufgebaut ist zu Figur 8, Funktionsweise siehe oben) Bestandteile des Positionierungssystems 122. Somit lassen sich zusätzliche Informationen über die Positionierung generieren. Beispielsweise lässt sich mittels der Kamera 910 der fokussierte Spot des von der Anregungs-Leuchtdiode 820 generierten Anregungslichts auf dem Okularsensor 116 beobachten, die korrekte Positionierung (beispielsweise eine konzentrische Positionierung innerhalb einer kreisrunden Augenlinse 210) bestimmen und entsprechend eine Messung des Messsystems 120 auslösen. Zusätzlich kann die Intensitätsmessung durch die Piloteinheit 812 berücksichtigt werden. Die Intensitäten können beispielsweise auch nacheinander ermittelt werden, so dass beispielsweise zunächst mittels der Piloteinheit 812 ein Pilotsignal zur Grobpositionierung erzeugt wird, um anschließend mittels der Kamera 910 eine Feinpositionierung vornehmen zu können. Derartige Systeme weisen eine hohe Präzision auf und führen dementsprechend zu sehr gut reproduzierbaren Messungen der Analytkonzentration.

Analog zur Beschreibung gemäß Figur 8 lässt sich das in Figur 9 gezeigte System dadurch modifizieren, dass zur Anregung von Donor und Fluoreszenzreferenz unterschiedliche Anregungs-Leuchtdioden verwendet werden. Dementsprechend könnte dem Aufbau der Messeinheit 810 eine weitere Anregungs-Leuchtdiode hinzugefügt werden, welche auf die Anregungswellenlänge des Referenzfluorophors im Okularsensor 116 abgestimmt ist. Auch ein weiterer dichroitischer Spiegel käme in diesem Fall vorteilhafter Weise hinzu. Auf diese Weise müssen Referenzfluorophor und Donor nicht notwendigerweise so gewählt werden, dass diese eine gemeinsame Anregungswellenlänge besitzen, was die Flexibilität bezüglich der Auswahl dieser Materialien zusätzlich erhöht.

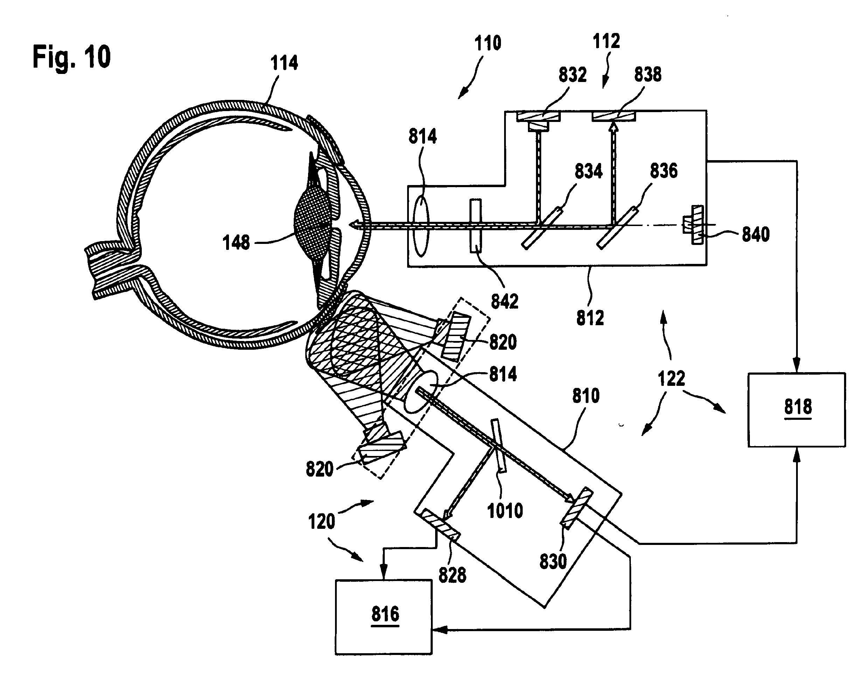

In Figur 10 ist eine zu den Figuren 8 und 9 alternative Modifikation des analytischen Messsystems 110 dargestellt. Die Piloteinheit 812 entspricht wiederum den Ausgestaltungen in den Figuren 8 und 9, so dass auf die obige Beschreibung verwiesen werden kann. Im Gegensatz zu den vorhergehenden Ausführungsbeispielen ist jedoch im Ausführungsbeispiel gemäß Figur 10 die Messeinheit 810 dahingehend modifiziert, dass anstelle einer einzelnen Anregungs-Leuchtdiode 820 nunmehr ein um die Linse 814 der Messeinheit 810 angeordneter Kranz von Anregungs-Leuchtdioden 820 eingesetzt wird. Deren Anregungslicht wird nun nicht mehr, wie in den Figuren 8 und 9, durch die Linse 814 gebündelt und auf den Okularsensor 116 fokussiert. Dementsprechend ist der Messfleck beziehungsweise der Anregungsbereich im Auge 114 in der Regel größer als die Implantat-Augenlinse 210, so dass die Augenlinse 210 von ihrem Durchmesser her sehr klein gestaltet sein kann. Dementsprechend ist die Positionierung des Implantats bei der Implantierungsoperation nicht so kritisch wie beispielsweise in den Ausführungsbeispielen gemäß den Figuren 8 und 9. Auch hat es sich gezeigt, dass die Messung der Analytkonzentration bei einem derartigen Aufbau unkritischer reagiert auf die exakte Positionierung des Handmessgeräts 112.

Entsprechend dem kranzförmigen Aufbau der Anregungs-Leuchtdioden 820 außerhalb der Linse 814 vereinfacht sich auch der innere optische Aufbau der Messeinheit 810. Anstelle von mindestens zwei dichroitischen Spiegeln ist nunmehr lediglich ein dichroitischer Spiegel 1010 vorgesehen, welcher das Fluoreszenzlicht des Donors vom Fluoreszenzlicht des Referenzfluorophors trennt. Die Aufnahme dieser Fluoreszenzkomponenten und deren Messung erfolgt entsprechend den Figuren 8 und 9 mittels der Photodioden 828 bzw. 830. Die weitere Auswertung der Messung beziehungsweise das Messprinzip folgt entsprechend dem Aufbau in Figur 8.

Auch in Figur 10 kann wiederum - anstelle einer Anregung mittels eines einzigen Wellenlängenbereichs - eine getrennte Anregung von Donor und Referenzfluorophor erfolgen. Dementsprechend können beispielsweise neben der Anregungs-Leuchtdiode 820 weitere Anregungs-Leuchtdioden vorgesehen sein, welche speziell auf die Anregungswellenlänge des Referenzfluorophors abgestimmt sind. Beispielsweise kann in der ringförmigen Anordnung der Leuchtdioden 820 um die Linse 814 herum jede zweite Leuchtdiode auf die Anregungswellenlänge des Referenzfluorophors abgestimmt sein.

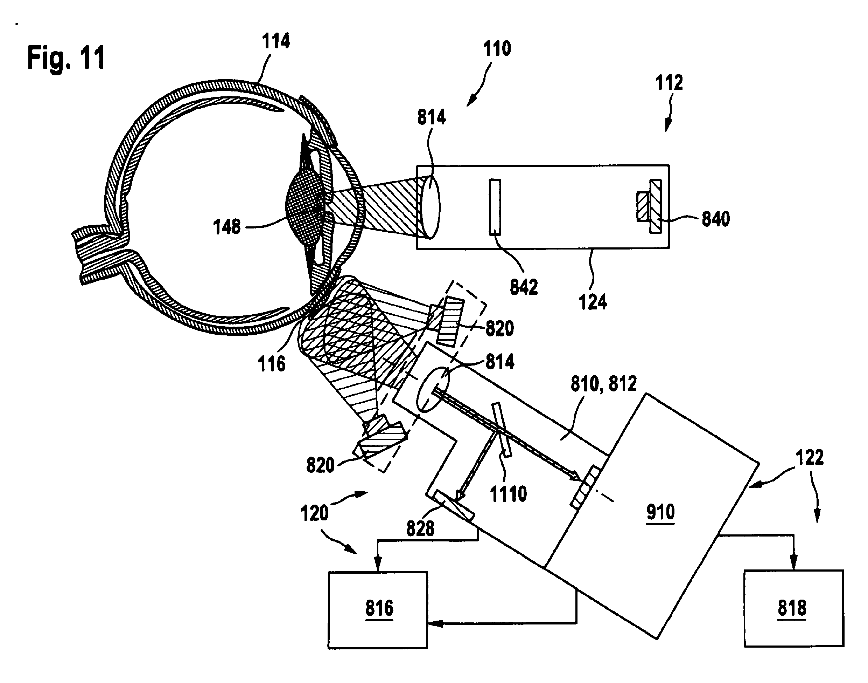

In Figur 11 ist ein Ausführungsbeispiel eines analytischen Messsystems 110 dargestellt, welches die Grundgedanken der Ausführungsbeispiele der Figuren 9 und 10 kombiniert. In diesem Ausführungsbeispiel werden die Funktionen von Messeinheit 810 und Piloteinheit 812 von derselben Einheit wahrgenommen. Analog zur Ausführung in Figur 10 ist die Messeinheit 810 wieder mit einem Kranz von Anregungs-Leuchtdioden 820 ausgestattet, welche den Bereich des als Augenlinse 210 ausgestalteten Okularsensors 116 großflächig bestrahlen. Vom Donor emittiertes Fluoreszenzlicht wird analog zur Ausgestaltung in Figur 10 vom dichroitischen Spiegel 1110 reflektiert und von der Donor-Photodiode 828 registriert.

Im Gegensatz zur Figur 10 ist jedoch - analog zur Ausgestaltung in Figur 9 - die Referenz-Photodiode (830 in Figur 10) ersetzt durch eine Kamera 910. Wie oben beschrieben, kann mittels dieser Kamera nicht nur die Intensität der Referenzfluoreszenz ermittelt werden (welche dann, gemeinsam mit dem Signal der Donor-Photodiode 828 von der Messauswertung 816 zur Bestimmung der Analytkonzentration verwendet wird), sondern es kann auch - analog zur obigen Beschreibung der Funktionsweise des Aufbaus in Figur 9 - eine Bildauswertung des Signals der Kamera 910 erfolgen. Diese Bildauswertung generiert eine ausreichende Positionierungsinformation, um eine genaue Messung zu ermöglichen. Dies ist insbesondere dadurch bedingt, dass bei der großflächigen Beleuchtung durch die Anregungs-Leuchtdioden 820 - wie oben beschrieben - eine geringere Positionsempfindlichkeit der Bestimmung der Analytkonzentration auftritt als in Beispielen, in welchen eine exakte Fokussierung erfolgt.

Daneben ist in dem Ausführungsbeispiel gemäß Figur 11 in das Handmessgerät 112 noch die Feedback-Einheit 124 integriert. Diese Feedback-Einheit 124 weist wiederum das transparente Anzeigenelement 842 und das Backlight 840 auf, wobei das transparente Anzeigenelement 842, wie oben anhand von Figur 8 beschrieben, beispielsweise mit Informationen des Positionierungssystems 822 beaufschlagt werden kann. Auf diese Weise kann mit Hilfe beispielsweise eines Fadenkreuzes beziehungsweise Retikels im transparenten Anzeigenelement 842 eine Grobpositionierung des Handmessgeräts 112 durch den Patienten erfolgen, bevor dann mittels des Positionierungssystems 122 die räumliche Positionierung weiter gemessen wird, entsprechende Korrektursignale erzeugt werden und/oder eine Messung des Messsystems 120 ausgelöst wird.

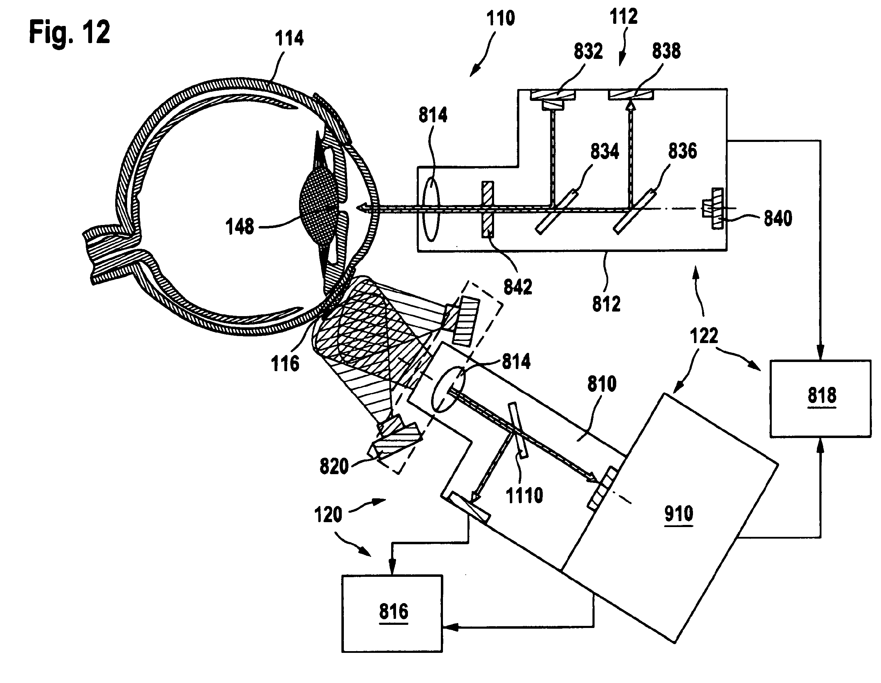

In Figur 12 ist ein Ausführungsbeispiel des analytischen Messsystems 110 dargestellt, welches eine Messeinheit 810 gemäß dem Ausführungsbeispiel in Figur 11 mit einer Piloteinheit 812 gemäß dem Ausführungsbeispiel in Figur 8 kombiniert. Für den Aufbau und die Funktionsweise dieser Einheiten sei auf diese Figuren verwiesen. Dementsprechend kann zum Beispiel zunächst eine Grobpositionierung mittels der Piloteinheit 812 vorgenommen werden, woraufhin dann eine Feinpositionierung mittels der Kamera 910 vorgenommen wird. Auch eine gleichzeitige Verarbeitung der Informationen der Piloteinheit 812 und der Kamera 910 zur Gewinnung von Positionierungsinformationen durch die Positionierungsauswertung 818 ist möglich. So kann beispielsweise mittels der Piloteinheit 812 eine Abstandsmessung durchgeführt werden, wohingegen dann mittels der Kamera 910 eine Positionierungsmessung mit zusätzlichen Positionierungskoordinaten erfolgt. Wiederum kann das transparente Anzeigenelement 842 für Benutzerinformationen (Feedback) genutzt werden.

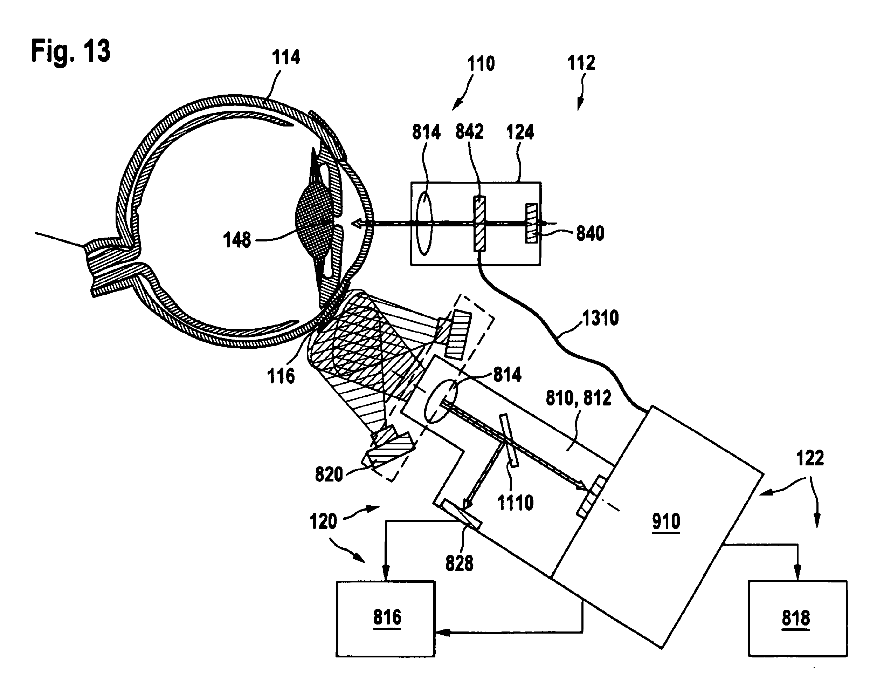

In Figur 13 ist ein Ausführungsbeispiel eines analytischen Messsystems 110 dargestellt, welches in seiner Funktion und in seinem Aufbau weitgehend dem Ausführungsbeispiel gemäß Figur 11 entspricht. Wiederum werden hier Messeinheit 810 und Piloteinheit 812 in derselben Einheit integriert. Auch ist wiederum eine Feedback-Einheit 124 vorgesehen, welche dem Patienten Informationen über die Positionierung liefert und welche neben einer Linse 814 und einem Backlight 840 ein transparentes Anzeigenelement 842 aufweist. Wie auch in Figur 11 kann auch in dem Aufbau gemäß Figur 13 über das transparente Anzeigenelement 842 beispielsweise ein Fadenkreuz für die Grobpositonierung eingeblendet werden. Zusätzlich wird jedoch über ein Bildkabel 1310 unmittelbar das Kamerabild der Kamera 910 auf das transparente Anzeigenelement 842 eingespielt, so dass der Benutzer direkt die Bildinformation erhält, welche auch das Positionierungssystem 122 "sieht". Beispielsweise kann dann eine Fixierung des Auges 114 mittels des Backlights 840 und des transparenten Anzeigenelements 842 erfolgen, wohingegen eine Einstellung des optimalen Abstandes des Handmessgeräts 112 zum Auge 114 beispielsweise über die Bildschärfe (zum Beispiel die Schärfe der Abbildung des Okularsensors 116) erfolgen kann. Wiederum kann durch das Positionierungssystem 122 dann automatisch eine Messung durch das Messsystem 120 ausgelöst werden, sobald ein optimaler Abstand erreicht ist und zumindest eine weitere Positionierungskoordinate in einem vorgegebenen Bereich liegt. Eine Rückkopplung der Informationen kann an den Patienten unmittelbar über das Kamerabild mittels des transparenten Anzeigenelements 842 erfolgen. Weiterhin können noch zusätzliche Informationen eingeblendet werden, wie Messergebnisse, Plausibilität der Messung, Datum, Zeit, Temperatur etc.

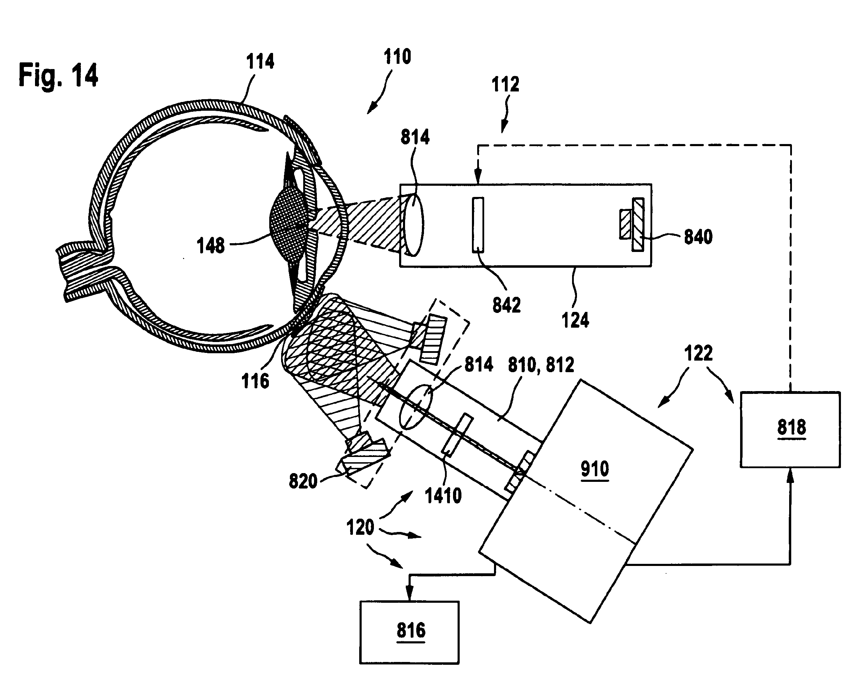

In Figur 14 ist ein Ausführungsbeispiel eines analytischen Messsystems 110 dargestellt, welches Ähnlichkeiten mit dem Aufbau gemäß Figur 11 aufweist. Wiederum ist eine Feedback-Einheit 124 mit einem Backlight 840 und einem transparenten Anzeigenelement 842 vorgesehen, welches mit Informationen aus der Positionierungsauswertung 818 versorgt werden kann. Auch erfolgt analog zur Ausführung in Figur 11 die Positionierungsmessung mit einer Kamera 910.

Im Unterschied zur Ausführung gemäß Figur 11 ist jedoch in dem Ausführungsbeispiel gemäß Figur 14 keine separate Donor-Photodiode 828 vorgesehen. Statt dessen erfolgt auch die Auswertung des Donor-Fluoreszenzlichts mit der Kamera 910. Zur Trennung zwischen Donor- Fluoreszenzlicht und Referenzfluoreszenzlicht kann beispielsweise ein Filter 1410 vorgesehen sein, welcher beispielsweise sequentiell die Fluoreszenzwellenlänge des Donors bzw. des Referenzfluorophors hindurchlässt. Auch Filter mit räumlich unterschiedlichen Transmissionseigenschaften sind denkbar. Auf diese Weise lassen sich räumlich und/oder zeitlich aufgelöst die Intensitäten der Referenzfluoreszenz und der Donor-Fluoreszenz messen.

Der Vorteil des Aufbaus gemäß Figur 14 besteht darin, dass der implantierte Okularsensor 116 sehr klein ausgebildet sein kann. Eine Positionierung des Implantats ist bei der Operation nicht so kritisch wie bei einer fokussierenden Optik. Gleichzeitig besteht eine geringere Abstandsempfindlichkeit der Messgenauigkeit als bei anderen Aufbauten. Zudem können die Positionierung und die Messung mit einem einzigen Sensor, nämlich der Kamera 910 erfolgen, so dass keine Bauteiltoleranzen unterschiedlicher Sensoren zueinander zu berücksichtigen bzw. durch eine Kalibrierung zu ermitteln sind. Weiterhin ist eine Positionierung (zum Beispiel eine Zentrierung) der Implantat-Abbildung auf dem als Sensor fungierenden CMOS-Chip der Kamera 910 nicht erforderlich, da eine größere Messfläche zur Verfügung steht.

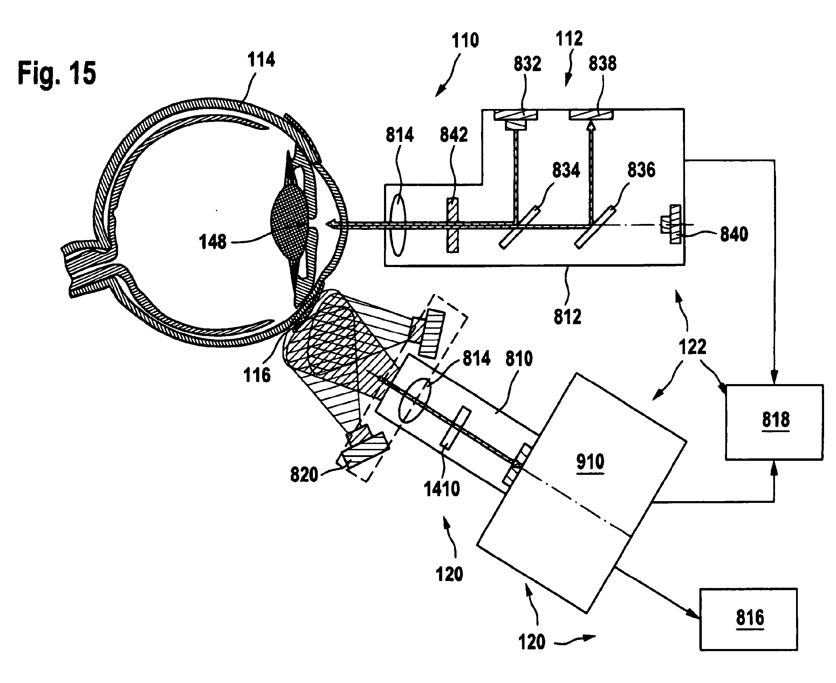

In Figur 15 ist ein Ausführungsbeispiel des analytischen Messsystems 110 dargestellt, welches den Aufbau der Messeinheit 810 gemäß dem Ausführungsbeispiel in Figur 14 mit dem Aufbau der Piloteinheit 812 gemäß dem Ausführungsbeispiel in Figur 8 kombiniert. Für die Funktionsweise und den Aufbau dieser Einheiten 810, 812 sei dementsprechend auf diese Figuren verwiesen.

Im Gegensatz zum Aufbau gemäß Figur 14 ist es mit dem analytischen Messsystem 110 gemäß Figur 15 möglich, eine Abstandsmessung mit der Piloteinheit 812 vorzunehmen, um diese dann durch zusätzliche Positionierungsinformationen der Kamera 910 zu kombinieren. Wiederum kann das transparente Anzeigenelement 842 zur Rückkopplung bei der Feinjustage verwendet werden.

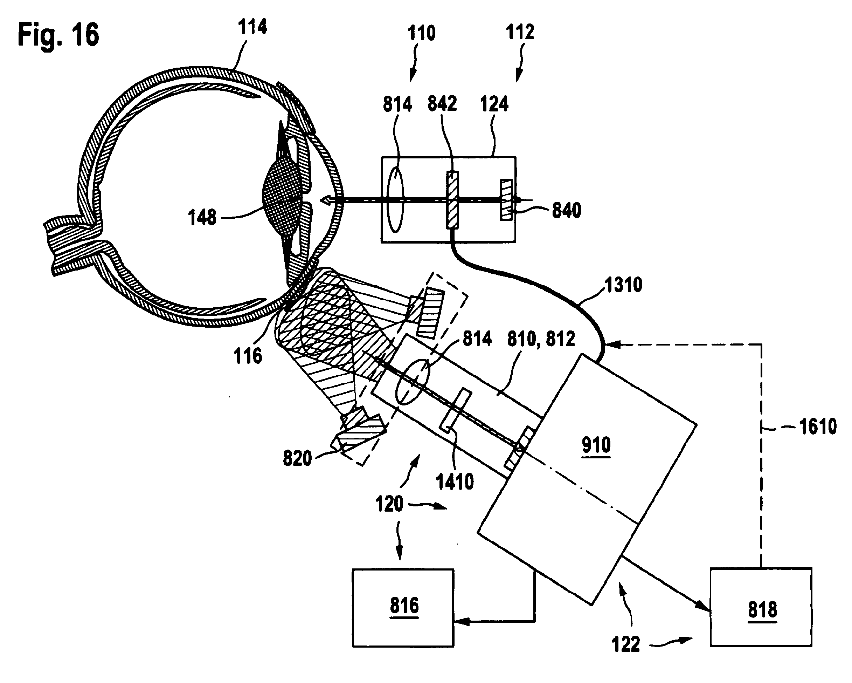

In Figur 16 ist schließlich ein Ausführungsbeispiel des analytischen Messsystems 110 dargestellt, bei welchem das Prinzip der integrierten Messeinheit, Piloteinheit 810, 812 gemäß dem Ausführungsbeispiel in den Figuren 14 und 15 kombiniert wird mit dem Prinzip der Feedback-Einheit 124 gemäß dem Ausführungsbeispiel in Figur 13. Dementsprechend ist wiederum ein Bildkabel 1310 zwischen Kamera 910 und Feedback-Einheit 124 vorgesehen, mittels dessen das Bild der Kamera 910 unmittelbar auf das transparente Anzeigenelement 842 (beispielsweise einen Flüssigkristall-Bildschirm) übertragen werden kann. Diese Bildübertragung kann - wie durch den gestrichelten Pfeil 1610 angedeutet - durch die Positionierungsauswertung 818 oder durch das gesamte Positionierungssystem 122 gesteuert werden.

Bezugszeichenliste

| | |
|---|---|
| 110 | analytisches Messsystem |
| 112 | Handmessgerät |
| 114 | Auge |
| 116 | Okularsensor |
| 118 | Positionierungssensor |
| 120 | Messsystem |
| 122 | Positionierungssystem |
| 124 | Feedback-Einheit |
| 126 | Feedback-Signal |
| 128 | Kalibriersystem |
| 130 | Bedieneinheit |
| 132 | optische Anzeigeelemente |
| 134 | Bedienelemente |
| 136 | zentrale Recheneinheit |
| 138 | Piezosteuerung |
| 140 | Blutstropfen |
| 142 | Teststreifen |
| 144 | Messsignale |
| 146 | Positionierungssignale |
| 148 | Pupille |
| 210 | Augenlinse |
| 212 | Limbus |
| 214 | Conjunctiva |
| 410 | Kamera |
| 412 | Körper |
| 510 | erste Position |
| 512 | zweite Position |
| 710 | Magnetisierung |
| 810 | Messeinheit |
| 812 | Piloteinheit |
| 814 | Linse |
| 816 | Messauswertung |
| 818 | Positionierungsauswertung |
| 820 | Anregungs-Leuchtdiode |
| 822 | dichroitischer Spiegel |
| 824 | dichroitischer Spiegel |
| 826 | dichroitischer Spiegel |
| 828 | Donor-Photodiode |
| 830 | Referenz-Photodiode |
| 832 | Pilot-Leuchtdiode |
| 834 | dichroitischer Spiegel |

836 dichroitischer Spiegel

838 Pilot-Photodiode

840 Backlight

842 transparentes Anzeigenelement

1010 dichroitischer Spiegel

1110 dichroitischer Spiegel

1310 Bildkabel

1410 Filter

1610 Steuerung der Bildübertragung

**Patentansprüche**

1. Handmessgerät (112) zur Messung mindestens eines Analyten in einer Augenflüssigkeit eines Auges (114), mit

   - einem Messsystem (120), welches eingerichtet ist, um mindestens eine Eigenschaft des mindestens einen Analyten und/oder mindestens eine Analyt-bedingte Eigenschaftsänderung mindestens eines Okularsensors (116) in der Augenflüssigkeit zu messen, und
   - einem Positionierungssystem (122), welches eingerichtet ist, um eine räumliche Positionierung zu messen, wobei die räumliche Positionierung einen Abstand zwischen mindestens einem Messort des Auges (114) und dem Handmessgerät (112) sowie weiterhin mindestens eine weitere Positionierungskoordinate umfasst,

   **dadurch gekennzeichnet, dass** das Positionierungssystem (122) mindestens eines der folgenden Systeme umfasst: ein Kamerasystem, insbesondere ein monokulares oder binokulares Kamerasystem, mit mindestens einer Kamera (410; 910); ein Bilderkennungssystem; ein Triangulationssystem mit zwei Kameras (410); ein Laufzeitmesssystem, insbesondere für eine 1-, 2- oder 3- dimensionale Laufzeitmessung, insbesondere mittels mindestens eines Lasers und/oder mindestens eines Phasenmischdetektors (PMD); eine 1-, 2- oder 3-dimensionale Intensitätsmesssystem mindestens eines Signals eines reflektierten divergenten elektromagnetischen und/oder akustischen Strahls; ein 2- oder 3-dimensionales magnetoresistives Messsystem.

2. Handmessgerät (112) gemäß dem vorhergehenden Anspruch, wobei das Messsystem (120) mindestens eines der folgenden Systeme umfasst: ein Infrarot (IR)-spektroskopisches Messsystem, ein Nah-Infrarot (NIR)-spektroskopisches Messsystem, ein RAMAN-spektroskopisches Messsystem, ein UV/sichtbar (UV/VIS)-spektroskopisches Messsystem, ein Fluoreszenzmesssystem, ein Impedanz-Messsystem, ein photoakustisches Messsystem, ein zirkular-dichroitisches Messsystem, ein refraktometrisches Messsystem, ein interferometrisches Messsystem.

3. Handmessgerät (112) gemäß einem der beiden vorhergehenden Ansprüche, wobei die mindestens eine weitere Positionierungskoordinate mindestens eine der folgenden Größen umfasst: einen Winkel einer virtuellen Verbindungslinie zwischen dem Handmessgerät (112) und dem mindestens einen Messort in einem vorgegebenen Winkelsystem, eine Koordinate des Handmessgeräts (112), eine Koordinate des mindestens einen Messorts, einen Ausrichtungswinkel des Handmessgeräts (112) in einem vorgegebenen Koordinatensystem.

4. Handmessgerät (112) gemäß einem der vorhergehenden Ansprüche, wobei das Positionierungssystem (122) ein Messsystem zum Vergleich mindestens zweier mittels zweier unterschiedlich räumlich angeordneter Sensoren (830, 838, 910) gemessener Signale umfasst.

5. Handmessgerät (112) gemäß einem der vorhergehenden Ansprüche, wobei das Positionierungssystem (122) bei Erreichen mindestens einer vorgegebenen Sollpositionierung oder eines vorgegebenen Sollpositionierungsbereichs eine Messung des Messsystems (120) auslöst.

6. Handmessgerät (112) gemäß einem der vorhergehenden Ansprüche, wobei das Positionierungssystem (122) eine räumliche Position und/oder räumliche Ausrichtung des Messsystems (120) einstellt.

7. Handmessgerät (112) gemäß dem vorhergehenden Anspruch, wobei eine Piezosteuerung (138) zur Einstellung der räumlichen Position und/oder der räumlichen Ausrichtung des Messsystems (120) vorgesehen ist.

8. Handmessgerät (112) gemäß einem der vorhergehenden Ansprüche, wobei das Positionierungssystem (122) und/oder eine Feedback-Einheit (124) des Handmessgeräts (112) eingerichtet sind, um entsprechend der räumlichen Positionierung mindestens ein Feedback-Signal (126) für einen Benutzer zu erzeugen.

9. Handmessgerät (112) gemäß einem der vorhergehenden Ansprüche, wobei das Handmessgerät (112) eingerichtet ist, um eine Bestimmung der Konzentration des mindestens einen Analyten in der Augenflüssigkeit und/oder in einer anderen Körperflüssigkeit, insbesondere in Blut oder Gewebeflüssigkeit, durchzuführen.

10. Handmessgerät (112) gemäß dem vorhergehenden Anspruch, wobei das Handmessgerät (112) eingerichtet ist, um bei der Bestimmung der mindestens einen Analytkonzentration die räumliche Positionierung zu berücksichtigen.

11. Handmessgerät (112) gemäß einem der beiden vorhergehenden Ansprüche, mit zusätzlich einem Kalibriersystem (128), wobei das Kalibriersystem (128) eingerichtet ist, um eine Vergleichsmessung mindestens einer Analytkonzentration in einer Körperflüssigkeit durchzuführen und/oder Messdaten einer mittels eines separaten Messgerätes durchgeführten Vergleichsmessung zu übernehmen und bei der Bestimmung der Konzentration des mindestens einen Analyten zu berücksichtigen.

12. Analytisches Messsystem (110) zur Messung mindestens eines Analyten in einer Augenflüssigkeit, umfassend ein Handmessgerät (112) gemäß einem der vorhergehenden Ansprüche sowie weiterhin mindestens einen Okularsensor (116), welcher geeignet ist, in Kontakt mit der Augenflüssigkeit gebracht zu werden, wobei der mindestens eine Okularsensor (116) ausgestaltet ist, um bei Kontakt mit dem mindestens einen Analyten mindestens eine Eigenschaft zu ändern, wobei die mindestens eine Eigenschaftsänderung mit dem Messsystem (120) messbar ist.

13. Analytisches Messsystem (110) gemäß dem vorhergehenden Anspruch, wobei der mindestens eine Okularsensor (116) mindestens eines der folgenden Elemente umfasst: eine Augenlinse (210), insbesondere eine Kontaktlinse; ein Augenimplantat.

14. Analytisches Messsystem (110) gemäß einem der beiden vorhergehenden Ansprüche, wobei der mindestens eine Okularsensor (116) mindestens einen Analyt-Rezeptor mit mindestens einem erstem Fluoreszenzlabel und mindestens einen Analyt-Konkurrenten mit mindestens einem zweitem Fluoreszenzlabel aufweist, wobei der mindestens eine Analyt-Rezeptor und der mindestens eine Analyt-Konkurrent ausgestaltet sind, um bei Bindung des mindestens einen Analyt-Konkurrenten an den mindestens einen Analyt-Rezeptor mindestens eine Eigenschaft des Okularsensors, (116) insbesondere mindestens eine Fluoreszenzeigenschaft, zu ändern.

15. Analytisches Messsystem (110) gemäß einem der vorhergehenden, auf ein analytisches Messsystem (110) gerichteten Ansprüche, wobei der mindestens eine Okularsensor (116) mindestens ein Gitter und/oder mindestens ein Hologramm aufweist, wobei das mindestens eine Gitter und/oder das mindestens eine Hologramm ausgestaltet sind, um bei Kontakt mit dem mindestens einen Analyten mindestens eine Reflexionseigenschaft zu ändern.

16. Analytisches Messsystem (110) gemäß einem der vorhergehenden, auf ein analytisches Messsystem (110) gerichteten Ansprüche, wobei der mindestens eine Okularsensor (116) weiterhin mindestens ein von dem mindestens einen Analyten zumindest im Wesentlichen unbeeinflussbaren Referenzfluorophor und/oder einen Referenzfarbstoff aufweist.

17. Analytisches Messsystem (110) gemäß einem der vorhergehenden, auf ein analytisches Messsystem (110) gerichteten Ansprüche, wobei der mindestens eine Analyt mindestens einen der folgenden Stoffe aufweist: Glucose, ein Hormon.

18. Analytisches Messsystem (110) gemäß einem der vorhergehenden, auf ein analytisches Messsystem (110) gerichteten Ansprüche mit zusätzlich mindestens einem Positionierungssensor (118), wobei der mindestens eine Positionierungssensor (118) getrennt von dem mindestens einen Okularsensor (116) ist oder Bestandteil des mindestens einen Okularsensors (116) ist und wobei der mindestens eine Positionierungssensor (118) ausgestaltet ist, um mindestens ein von dem Positionierungssystem (122) wahrnehmbares Signal (146) zu erzeugen.

19. Analytisches Messsystem (110) gemäß dem vorhergehenden Anspruch, wobei der mindestens eine Positionierungssensor (118) mindestens eines der folgenden Elemente umfasst: eine Augenlinse (210), insbesondere eine Kontaktlinse; ein Augenimplantat.

20. Analytisches Messsystem (110) gemäß einem der beiden vorhergehenden Ansprüche, wobei das mindestens eine von dem Positionierungssystem (122) wahrnehmbare Signal (146) mindestens eines der folgenden Signale umfasst: eine wahrnehmbare Gestalt oder Markierung; eine Fluoreszenz; eine Magnetisierung (710); ein Referenzfluoreszenzsignal; ein Referenzfarbsignal.

21. Verfahren zur Bestimmung einer Konzentration mindestens eines Analyten in einer Körperflüssigkeit unter Verwendung eines analytischen Messsystems (110) gemäß einem der vorhergehenden, auf ein analytisches Messsystem (110) gerichteten Ansprüche, mit folgenden Schritten:

   a) das Handmessgerät (112) wird vor dem Auge (114) grob positioniert,

b) eine räumliche Positionierung wird bestimmt,

c) eine Messung des Messsystems (120) wird ausgelöst,

d) aus der mindestens einen gemessenen Eigenschaft des mindestens einen Analyten und/oder der mindestens einen gemessenen Eigenschaftsänderung des mindestens einen Okularsensors (116) wird die Konzentration des mindestens einen Analyten in der Augenflüssigkeit bestimmt, und

e) die Konzentration des mindestens einen Analyten in der Augenflüssigkeit wird mittels eines vorgegebenen Umrechnungsalgorithmus in eine Konzentration des mindestens einen Analyten in der Körperflüssigkeit umgerechnet.

**Claims**

1. Handheld instrument (112) for the measurement of at least one analyte in an eye fluid of an eye (114), having

   - a measurement system (120) which is designed in order to measure at least one characteristic of the at least one analyte and/or at least one analyte-dependent characteristic change of at least one ocular sensor (116) in the eye fluid and
   - a positioning system (122) which is designed in order to measure a spatial position, with this spatial position comprising a distance between at least one measurement point of the eye (144) and the handheld instrument (112) and furthermore at least one further position coordinate,

   **characterized in that** the positioning system (122) comprises at least one of the following systems: a camera system, in particular a monocular or binocular camera system, having at least one camera (410; 910); an image recognition system; a triangulation system with two cameras (410); a propagation time measurement system, in particular for a one-dimensional, two-dimensional or three-dimensional propagation time measurement, in particular by means of at least one laser and/or at least one phase mixing detector (PMD); a one-dimensional, two-dimensional or three-dimensional intensity measurement system for at least one signal of a reflected divergent electromagnet and/or acoustic beam; a two-dimensional or three-dimensional magnetoresistive measurement system.

2. Handheld instrument (112) according to the preceding claim, with the measurement system (120) comprising at least one of the following systems: an infrared (IR)-spectroscopic measurement system, a near-infrared (NIR)-spectroscopic measurement system, a RAMAN-spectroscopic measurement system, a UV/visible (UV/VIS)-spectroscopic measurement system, a fluorescence measurement system, an impedance measurement system, a photoacoustic measurement system, a circular-dichroic measurement system, a refractometric measurement system, an interferometric measurement system.

3. Handheld instrument (112) according to one of the two preceding claims, with the at least one further position coordinate comprising at least one of the following variables: an angle of a virtual connecting line between the handheld instrument (112) and the at least one measurement point in a predetermined angle system, a coordinate of the handheld instrument (112), a coordinate of the at least one measurement point, an alignment angle of the handheld instrument (112) in a predetermined coordinate system.

4. Handheld instrument (112) according to one of the preceding claims, with the positioning system (112) comprising a measurement system for comparison of at least two signals which are measured by means of two sensors (830, 838, 910) which are arranged spatially differently.

5. Handheld instrument (112) according to one of the preceding claims, with the positioning system (122) initiating a measurement of the measurement system (120) on reaching at least one predetermined nominal position or a predetermined nominal position range.

6. Handheld instrument (112) according to one of the preceding claims, with the positioning system (122) setting a spatial position and/or spatial alignment of the measurement system (120).

7. Handheld instrument (112) according to the preceding claim, with a piezo-control (138) being provided in order to set the spatial position and/or the spatial alignment of the measurement system (120).

8. Handheld instrument (112) according to one of the preceding claims, with the positioning system (122) and/or a feedback unit (124) of the handheld instrument (112) being designed in order to produce at least one feedback signal (116) for a user, corresponding to the spatial position.

9. Handheld instrument (112) according to one of the preceding claims, with the handheld instrument (112) being designed in order to determine the concentration of the at least one analyte in the eye fluid and/or in some other bodily fluid, in particular in blood or tissue fluid.

10. Handheld instrument (112) according to the preceding claim, with the handheld instrument (112) being designed to take account of the spatial position when determining the at least one analyte concentration.

11. Handheld instrument (112) according to one of the two preceding claims, additionally having a calibration system (128), with the calibration system (128) being designed in order to carry out a comparative measurement of at least one analyte concentration in a bodily fluid and/or to receive measurement data from a comparative measurement carried out by means of a separate instrument, and to take this into account when determining the concentration of the at least one analyte.

12. Analytical measurement system (110) for the measurement of at least one analyte in an eye fluid, comprising a handheld instrument (112) according to one of the preceding claims and furthermore comprising at least one ocular sensor (116) which is suitable for being brought into contact with the eye fluid, with the at least one ocular sensor (116) being designed to change at least one characteristic on contact with the at least one analyte, and with the at least one characteristic change being measurable by means of the measurement system (120).

13. Analytical measurement system (110) according to the preceding claim, with the at least one ocular sensor (116) comprising at least one of the following elements: an eye lens (210), in particular a contact lens; an eye implant.

14. Analytical measurement system (110) according to one of the two preceding claims, with the at least one ocular sensor (116) having at least one analyte receptor with at least one first fluorescence label and at least one analyte competitor with at least one second fluorescence label, with the at least one analyte receptor and the at least one analyte competitor being designed to change at least one characteristic of the ocular sensor (116), in particular at least one fluorescence characteristic, on bonding of the at least one analyte competitor to the at least one analyte receptor.

15. Analytical measurement system (110) according to one of the preceding claims which relate to an analytical measurement system (110), with the at least one ocular sensor (116) having at least one grating and/or at least one hologram, with the at least one grating and/or the at least one hologram being designed to change at least one reflection characteristic on contact with the at least one analyte.

16. Analytical measurement system (110) according to one of the preceding claims which relate to an analytical measurement system (110), with the at least one ocular sensor (116) furthermore having at least one reference fluorogen which at least cannot be influenced significantly by the at least one analyte, and/or having a reference dye.

17. Analytical measurement system (110) according to one of the preceding claims which relate to an analytical measurement system (110), with the at least one analyte having at least one of the following substances: glucose, a hormone.

18. Analytical measurement system (110) according to one of the preceding claims which relate to an analytical measurement system (110), additionally having at least one position sensor (118), with the at least one position sensor (118) being separate from the at least one ocular sensor (116) or being a component of the at least one ocular sensor (116), and with the at least one position sensor (118) being designed to produce at least one signal (146) which can be perceived by the positioning system (122).

19. Analytical measurement system (110) according to the preceding claim, with the at least one position sensor (118) comprising at least one of the following elements: an eye lens (210), in particular a contact lens; an eye implant.

20. Analytical measurement system (110) according to one of the two preceding claims, with the at least one signal (146) which can be perceived by the positioning system (122) comprising at least one of the following signals: a perceptible form or marking; a fluorescence; a magnetization (710); a reference fluorescence signal; a reference colour signal.

21. Method for determination of a concentration of at least one analyte in a bodily fluid using an analytical measurement system (110) according to one of the preceding claims which relate to an analytical measurement system (110), having the following steps:

a) a handheld instrument (112) is positioned roughly in front of the eye (114),
b) a spatial position is determined,
c) a measurement of the measurement system (120) is initiated,
d) the concentration of the at least one analyte in the eye fluid is determined from the at least one measured characteristic of the at least one analyte and/or the at least one measured characteristic change of the at least one ocular sensor (116), and
e) the concentration of the at least one analyte in the eye fluid is converted by means of a predetermined conversion algorithm to a concentration of the at least one analyte in the bodily fluid.

## Revendications

1. Appareil de mesure portatif (112) pour la mesure d'au moins un analyte dans un fluide oculaire d'un oeil (114), avec

   - un système de mesure (120) qui est installé pour mesurer au moins une caractéristique d'au moins un analyte et/ou d'au moins une modification de caractéristique induite par l'analyte d'au moins un capteur oculaire (116) dans le fluide oculaire, et
   - un système de positionnement (122) qui est installé pour mesurer un positionnement dans l'espace, le positionnement dans l'espace comprenant une distance entre au moins un emplacement de mesure de l'oeil (114) et l'appareil de mesure portatif (112) ainsi en outre qu'une autre coordonnée de positionnement,

   **caractérisé en ce que** le système de positionnement (122) comprend au moins un des systèmes suivants : un système de caméra, notamment un système de caméra monoculaire ou binoculaire, avec au moins une caméra (410 ; 910) ; un système de reconnaissance d'images ; un système de triangulation à deux caméras (410) ; un système de mesure du temps de transit, notamment pour une mesure du temps de transit à 1, 2 ou 3 dimensions, notamment au moyen d'au moins un laser et/ou d'au moins un détecteur de mélange de phases (PMD) ; un système de mesure à 1, 2 ou 3 dimensions de l'intensité d'au moins un signal d'un faisceau électromagnétique et/ou acoustique divergent réfléchi ; un système de mesure magnéto-résistif à 2 ou 3 dimensions.

2. Appareil de mesure portatif (112) selon la revendication précédente, dans lequel le système de mesure (120) comprend au moins un des systèmes suivants : un système de mesure spectroscopique infrarouge (IR), un système de mesure spectroscopique dans l'infrarouge proche (NIR), un système de mesure spectroscopique RAMAN, un système de mesure spectroscopique UV/visible (UV/VIS), un système de mesure par fluorescence, un système de mesure par impédance, un système de mesure photo-acoustique, un système de mesure dichroïque circulaire, un système de mesure réfractométrique, un système de mesure interférométrique

3. Appareil de mesure portatif (112) selon une des deux revendications précédentes, dans lequel au moins une autre coordonnée de positionnement comprend au moins une des grandeurs suivantes : un angle d'une ligne de liaison virtuelle entre l'appareil de mesure portatif (112) et au moins un emplacement de mesure dans un système angulaire défini, une coordonnée de l'appareil de mesure portatif (112), une coordonnée d'au moins un emplacement de mesure, un angle d'orientation de l'appareil de mesure portatif (112) dans un système de coordonnées défini.

4. Appareil de mesure portatif (112) selon une des revendications précédentes, dans lequel le système de positionnement (122) comprend au moins un système de mesure pour la comparaison d'au moins deux signaux mesurés au moyen de deux capteurs (830, 838, 910) disposés à deux emplacements différents de l'espace.

5. Appareil de mesure portatif (112) selon une des revendications précédentes, dans lequel le système de positionnement (122) déclenche une mesure du système de mesure (120) lorsqu'il atteint au moins une position de consigne définie ou bien une plage de positionnements de consigne définie.

6. Appareil de mesure portatif (112) selon une des revendications précédentes, dans lequel le système de positionnement (122) règle une position dans l'espace et/ou une orientation dans l'espace du système de mesure (120).

7. Appareil de mesure portatif (112) selon la revendication précédente, dans lequel un pilotage piézoélectrique (138) est prévu pour le réglage de la position dans l'espace et/ou de l'orientation dans l'espace du système de mesure (120).

8. Appareil de mesure portatif (112) selon une des revendications précédentes, dans lequel le système de positionnement (122) et/ou une unité de feedback (124) de l'appareil de mesure portatif (112) sont installés pour engendrer pour un utilisateur, au moins un signal de feedback (126) en fonction du positionnement dans l'espace.

9. Appareil de mesure portatif (112) selon une des revendications précédentes, dans lequel l'appareil de mesure portatif (112) est installé pour déterminer la concentration d'au moins un analyte dans le fluide oculaire et/ou dans un autre fluide corporel, notamment dans le sang ou dans le fluide tissulaire.

10. Appareil de mesure portatif (112) selon la revendication précédente, dans lequel l'appareil de mesure portatif (112) est installé pour respecter le positionnement dans l'espace lors de la détermination d'au moins une concentration d'un analyte.

**11.** Appareil de mesure portatif (112) selon une des deux revendications précédentes, avec en plus un système de calibrage (128), dans lequel le système de calibrage (128) est installé pour effectuer une mesure comparative d'au moins une concentration d'analyte dans un fluide corporel et/ou pour prendre en charge des données de mesure d'une mesure comparative réalisée au moyen d'un appareil de mesure séparé et pour en tenir compte lors de la détermination de la concentration d'au moins un analyte.

**12.** Système de mesure analytique (110) pour la mesure d'au moins un analyte dans un fluide oculaire, comprenant un appareil de mesure portatif (112) selon une des revendications précédentes, avec de plus au moins un capteur oculaire (116) qui est apte à être amené au contact du fluide oculaire, cet au moins un capteur oculaire (116) est formé pour modifier au moins une caractéristique lors du contact avec au moins un analyte, cette au moins une modification de caractéristique étant mesurable par le système de mesure (120).

**13.** Système de mesure analytique (110) selon la revendication précédente, cet au moins un capteur oculaire (116) comprenant au moins un des éléments suivants : une lentille oculaire (210), notamment une lentille de contact ; un implant oculaire.

**14.** Système de mesure analytique (110) selon une des deux revendications précédentes, cet au moins un capteur oculaire (116) comportant au moins un récepteur d'analyte avec au moins un premier label de fluorescence et au moins un concurrent de l'analyte avec au moins un deuxième label de fluorescence, cet au moins un récepteur d'analyte et cet au moins un concurrent de l'analyte étant formés de telle sorte que lors de la liaison de cet au moins un concurrent de l'analyte avec cet au moins un récepteur d'analyte, au moins une caractéristique du capteur oculaire (116) se modifie, notamment au moins une caractéristique de fluorescence.

**15.** Système de mesure analytique (110) selon une des revendications précédentes concernant un système de mesure analytique (110), cet au moins un capteur oculaire (116) comportant au moins une grille et/ou au moins un hologramme, cette au moins une grille et/ou cet au moins un hologramme étant formés pour modifier au moins une caractéristique de réflexion lors du contact avec cet au moins un analyte.

**16.** Système de mesure analytique (110) selon une des revendications précédentes concernant un système de mesure analytique (110), cet au moins un capteur oculaire (116) comportant de plus au moins un fluorophore de référence et/ou un colorant de référence non influençable au moins pour l'essentiel par cet au moins un analyte.

**17.** Système de mesure analytique (110) selon une des revendications précédentes concernant un système de mesure analytique (110), cet au moins un analyte comportant au moins une des matières suivantes : glucose, une hormone.

**18.** Système de mesure analytique (110) selon une des revendications précédentes concernant un système de mesure analytique (110), avec en plus au moins un capteur de positionnement (118), cet au moins un capteur de positionnement (118) étant séparé de cet au moins un capteur oculaire (116) ou étant composant de cet au moins un capteur oculaire (116) et cet au moins un capteur de positionnement (118) étant formé pour générer au moins un signal (146) pouvant être enregistré par le système de positionnement (122).

**19.** Système de mesure analytique (110) selon la revendication précédente, dans lequel cet au moins un capteur de positionnement (118) comprend au moins un des éléments suivants : une lentille oculaire (210), notamment une lentille de contact ; un implant oculaire.

**20.** Système de mesure analytique (110) selon une des deux revendications précédentes, dans lequel cet au moins signal (146) pouvant être enregistré par le système de positionnement (122) comprend au moins un des signaux suivants : une forme ou un marquage pouvant être enregistrés ; une fluorescence, une magnétisation (710), un signal de fluorescence de référence, un signal de couleur de référence.

**21.** Procédé pour la détermination d'une concentration d'au moins un analyte dans un fluide corporel utilisant un système de mesure analytique (110) selon une des revendications précédentes concernant un système de mesure analytique (110), avec les étapes suivantes :

a) on positionne d'abord grossièrement l'appareil de mesure portatif (112) devant l'oeil (114),
b) on détermine un positionnement dans l'espace,
c) on déclenche une mesure du système de mesure (120),
d) on détermine la concentration de cet au moins un analyte dans le fluide oculaire à partir d'au moins une des caractéristiques mesurées de cet au moins un analyte et/ou d'au moins une modification des caractéristiques mesurée de cet au moins un capteur oculaire (116), et
e) on convertit la concentration de cet au moins un analyte dans le fluide oculaire en une con-

**EP 1 882 446 B1**

centration de cet au moins un analyte dans le fluide corporel au moyen d'un algorithme de conversion défini.

**Fig. 1**

EP 1 882 446 B1

# Fig. 2A

114

210

A

# Fig. 2B

212

D

214

210

EP 1 882 446 B1

# Fig. 3

114

210

D

# Fig. 4A

# Fig. 4B

## Fig. 4C

## Fig. 5A

Fig. 5B

## Fig. 6

## Fig. 7

Fig. 8

Fig. 9

EP 1 882 446 B1

Fig. 10

EP 1 882 446 B1

# Fig. 11

**Fig. 12**

Fig. 13

EP 1 882 446 B1

**Fig. 14**

Fig. 15

EP 1 882 446 B1

**Fig. 16**

EP 1 882 446 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 0113783 A
- WO 02087429 A
- WO 2004071287 A
- US 6122042 A
- WO 0113783 A1